# EUROPEAN PATENT APPLICATION

(11) **EP 4 666 923 A1**
(43) Date of publication of application: **24.12.2025**
(21) Application number: 24764208.5
(22) Date of filing: 28.02.2024
(51) Int. Cl.: A47L 7/00, A47L 9/20, A47L 9/28, A61L 2/04

(54) **VACUUM CLEANER STATION**

(30) Priority: 02.03.2023 KR 20230027808
(71) Applicant: LG Electronics Inc., Yeongdeungpo-gu Seoul 07336 (KR)
(72) Inventor: YANG, Ingyu, Seoul 08592 (KR); SHIN, Jinhyouk, Seoul 08592 (KR); JEONG, Yeonghan, Seoul 08592 (KR); LEE, Donggeun, Seoul 08592 (KR)
(74) Representative: Ter Meer Steinmeister & Partner
(86) International application number: PCT/KR2024/002610
(87) International publication number: WO 2024/181809

(57) **Abstract**

The present invention relates to a cleaner station and includes a housing, a coupling unit disposed in the housing and including a coupling surface to which at least a portion of a cleaner is coupled, a dust collection unit which is disposed in the housing and collects dust of a dust bin of the cleaner, a suction flow path unit connecting a dust through hole formed in the coupling surface to the dust collection unit, a dust collection motor which is accommodated inside the housing and generates a suction force of suctioning the dust inside the dust bin, and a heat circulation module that heats air suctioned from an internal space of the dust collection unit and supplies the heated air to the internal space, thereby maintaining a temperature of the dust collection unit within a predetermined range to maintain a clean and sanitary condition.

## Description

### [Technical Field]

The present invention relates to a cleaner station, and more particularly, to a cleaner station that maintains a clean and sanitary condition by maintaining a temperature of a dust collection unit within a predetermined range.

### [Background Art]

In general, a cleaner is a home appliance for suctioning small trash or dust in a manner of suctioning air using electricity and filling the same in a dust bin inside a product and is commonly called a vacuum cleaner.

The vacuum cleaner may be classified into a manual vacuum cleaner for allowing a user to directly perform cleaning while moving the cleaner, and an automatic vacuum cleaner for performing cleaning while traveling by itself. Depending on the type of the vacuum cleaner, the manual vacuum cleaner may be classified into a canister-type vacuum cleaner, an upright vacuum cleaner, a hand vacuum cleaner, a stick-type vacuum cleaner, etc.

In the past, the canister-type vacuum cleaner was widely used as the household vacuum cleaner, but recently, the hand vacuum cleaner and the stick-type vacuum cleaner, which provide improved convenience of use by integrally providing a dust bin and a cleaner body, are increasingly being used.

The canister-type vacuum cleaner has a main body and a suction port connected by a rubber hose or a pipe and in some cases, may be used by inserting a brush into the suction port.

The hand vacuum cleaner is designed to maximize portability and has lightweight and a short length, and thus can have a limited cleaning area. Accordingly, the hand vacuum cleaner is used to clean localized sites, such as on a desk, a sofa, or a vehicle interior.

A user may use the stick-type vacuum cleaner while standing to enable cleaning without bending down. Accordingly, it is advantageous in cleaning a wide area while moving. While the hand vacuum cleaner cleans narrow spaces, the stick-type vacuum cleaner may clean wider spaces and clean high places out of reach. Recently, the stick-type vacuum cleaner has been provided in a module type to allow users to actively change a vacuum cleaner type for various purposes.

However, since conventional handheld vacuum cleaners and the stick-type vacuum cleaners have a small capacity of dust bins that store collected dust, there is inconvenience that a user needs to empty the dust bin every time.

In addition, when the dust bin is emptied, there is a problem that dust flies and has a harmful effect on the user's health.

In addition, there is a problem that a suction force of the cleaner can be lowered and odors occur when the remaining dust in the dust bin is not removed.

As Patent Document, Korean Laid-Open Patent No. 10-2022-0027649 discloses a cleaner station that collects dust inside a dust bin of a cleaner.

The patent document discloses a dust storage module that collects dust by a dust collection motor, and a sterilization module that sterilizes the dust collected in the dust storage module, in which the dust storage module includes a transmissive panel through which sterilizing light emitted from the sterilization module passes.

However, in the patent document, the sterilization module is a component for emitting sterilizing light onto only portions of upper surfaces of foreign substances collected in the dust collection unit, and thus cannot sterilize central or lower portions of the foreign substances, onto which the sterilizing light is not emitted.

Accordingly, when unsterilized foreign substances remain in the central portion and the like of the dust collection unit for a long time, insects and microorganisms can proliferate. In particular, mites such as grain mites and the like, which are microorganisms parasitic on inadequately dried grains, can easily propagate in a hot and humid environment when grains are collected in the dust collection unit and move outside along a moving path of the cleaner station.

### [Disclosure]

### [Technical Problem]

The present invention is directed to providing a cleaner station capable of sterilizing insects and microorganisms, such as powdery mildew, contained in foreign substances introduced from a dust bin of the cleaner using high-temperature air.

In addition, the present invention is directed to providing a cleaner station capable of sterilizing insects and microorganisms that proliferate deep within foreign substances collected within a dust collection unit.

In addition, the present invention is directed to providing a cleaner station capable of maintaining a temperature of air of an internal space of a dust collection unit within a predetermined range.

In addition, the present invention is directed to providing a cleaner station capable of reducing a risk of ignition of oils and greases when sterilizing insects and microorganisms that proliferate within foreign substances containing oils and greases.

### [Technical Solution]

According to the present invention, there is provided a cleaner station including a housing, a coupling unit disposed in the housing and including a coupling surface to which at least a portion of a cleaner is coupled, a dust collection unit which is disposed in the housing and collects dust of a dust bin of the cleaner, a suction flow path unit connecting a dust through hole formed in the coupling surface to the dust collection unit, a dust collection motor which is accommodated inside the housing and generates a suction force of suctioning the dust inside the dust bin, and a heat circulation module that heats air suctioned from an internal space of the dust collection unit and supplies the heated air to the internal space.

The heat circulation module may include a circulation flow path unit having a suction port through which the air in the internal space is suctioned and a discharge port that discharges the suctioned air into the internal space, a circulation fan which is disposed on the circulation flow path unit and blows air from the suction port toward the discharge port, and a heater that heats the air blown by the circulation fan.

The dust collection unit may include a dust inlet that guides the air flowing through the suction port into the internal space, and the suction port and the discharge port may be disposed with the dust inlet interposed therebetween.

The heater may be disposed on the circulation flow path unit,
and a distance from the discharge port to the heater may be smaller than a distance from the discharge port to the circulation fan.

An open area of the discharge port may be larger than an open area of the suction port.

The heater may heat the air blown through the discharge port to 65 °C.

The temperature of the internal space may be 55 °C or higher and 65 °C or lower.

The heater may heat the air discharged into the internal space in a state in which the circulation fan is driven.

The circulation fan may be driven after the driving of the dust collection motor is terminated.

A deodorizing filter may be disposed in the discharge port.

The heat circulation module may include a suction port through which the air in the internal space is suctioned, a discharge port through which air is discharged into the internal space, a first circulation flow path disposed outside the dust collection unit and connecting the suction port to the discharge port, a second circulation flow path disposed in the internal space and connecting the suction port to the discharge port, a circulation fan which is disposed on the first circulation flow path and blows air from the suction port toward the discharge port, and a heater which heats the air blown by the circulation fan.

A cross-sectional area of the first circulation flow path may increase toward the discharge port.

A temperature of air flowing through the second circulation flow path may increase toward the discharge port.

A temperature of air flowing through the second circulation flow path may decrease toward the suction port.

A deodorizing filter may be disposed on the second circulation flow path.

### [Advantageous Effects]

As described above, the cleaner station according to the present invention can effectively sterilize insects and microorganisms introduced from the dust bin of the cleaner by discharging air heated to 65 °C through the heater into the dust collection unit.

In addition, according to the present invention, by generating heat that circulates in the internal space of the dust collection unit after dust collection is completed, insects and microorganisms that proliferate in the central portion and deep interior of foreign substances collected within the dust collection unit can be sterilized.

In addition, according to the present invention, by circulating the air in the internal space of the dust collection unit with the outside air, the air in the internal space of the dust collection unit can be maintained at the temperature of 55 °C or higher and 65 °C or lower.

In addition, according to the present invention, by effectively maintaining the temperature of the internal space of the dust collection unit at the temperature of 55 °C or higher and 65 °C or lower, it is possible to reduce the risk of ignition of oil/fat components when sterilizing insects and microorganisms that proliferate in foreign substances containing the oil/fat components.

### [Description of Drawings]

FIG. 1 is a perspective view of a cleaner system composed of a cleaner station and a cleaner according to an embodiment of the present invention.
FIG. 2 is a schematic diagram of a configuration of the cleaner system according to the embodiment of the present invention.
FIGS. 3 and 4 are views for describing a cleaner of the cleaner system according to the embodiment of the present invention.
FIG. 5 is a view for describing a lower surface of a dust bin of the cleaner according to the embodiment of the present invention.
FIG. 6 is a view for describing a coupling unit in the cleaner station according to the embodiment of the present invention.
FIG. 7 is an exploded perspective view for describing a fixing unit in the cleaner station according to the embodiment of the present invention.
FIGS. 8 and 9 are views for describing a relationship between the cleaner and a door unit in the cleaner station according to the embodiment of the present invention.
FIG. 10 is a view for describing the relationship between the cleaner and a cover opening unit in the cleaner system according to the embodiment of the present disclosure.
FIG. 11 is a block diagram for describing a control configuration of the cleaner system according to the embodiment of the present invention.
FIG. 12 is a cross-sectional view for describing a specific configuration of the cleaner station according to the embodiment of the present invention.
FIG. 13 is a perspective view for describing a heat circulation module of the cleaner station according to the embodiment of the present invention.
FIG. 14 is an exploded view of main components of a dust collection unit of the cleaner station according to the embodiment of the present invention.
FIG. 15 is an exploded view of main components of a dust storage module of the cleaner station according to the embodiment of the present invention.
FIG. 16 is an enlarged view of main components of a dust collection chamber of the cleaner station according to the embodiment of the present invention.
FIG. 17 is an exploded view for describing the heat circulation module of the cleaner station according to the embodiment of the present invention.
FIG. 18 is a cross-sectional view for describing the heat circulation module of the cleaner station according to the embodiment of the present invention.

### [Mode for Invention]

Hereinafter, exemplary embodiments of the present invention will be described in detail with reference to the accompanying drawings.

Since the present invention may have various changes and various embodiments, specific embodiments are shown in the accompanying drawings and specifically described in the detail descriptions. This is not intended to limit the present invention to specific embodiments and should be construed to include all modifications, equivalents, and substitutes included in the spirit and technical scope of the present invention.

The terms used in the present application are only used to describe specific embodiments and are not intended to limit the present invention. A singular expression includes a plural expression unless the context clearly dictates otherwise.

Unless defined otherwise, all terms used herein, including technical or scientific terms, have the same meaning as commonly understood by those skilled in the art to which the present invention pertains. The terms defined in a generally used dictionary can be construed as meanings that match with the meanings of the terms from the context of the related technology and are not construed as an ideal or excessively formal meaning unless clearly defined in the present application.

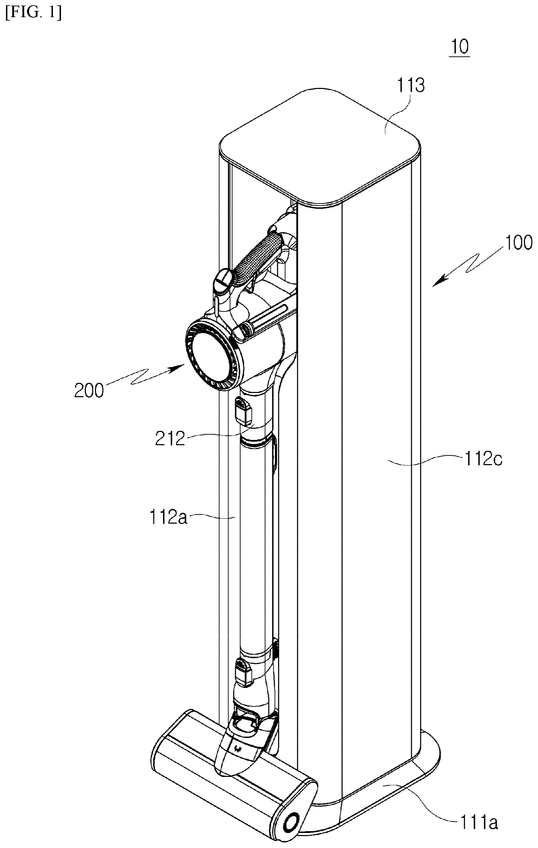

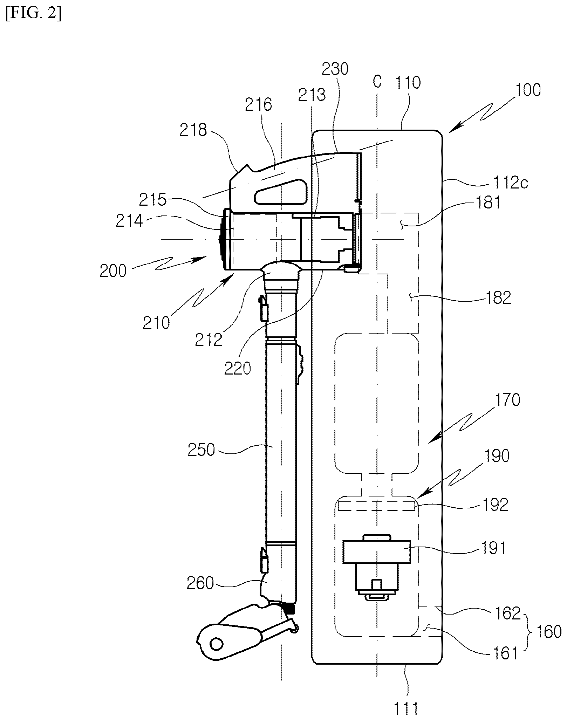

FIG. 1 shows a perspective view of a cleaner system composed of a cleaner station and a cleaner according to an embodiment of the present invention, and FIGS. 2 to 4 show schematic views of configurations of cleaner systems according to various embodiments of the present invention.

Referring to FIGS. 1 and 2, a cleaner system 10 according to an embodiment of the present invention may include a cleaner station 100 and a cleaner 200.

The cleaner system 10 may include the cleaner station 100. The cleaner 200 may be coupled to the cleaner station 100. Specifically, the main body of the cleaner 200 may be coupled to the side surface of the cleaner station 100. The cleaner station 100 may remove dust of a dust bin 220 of the cleaner 200.

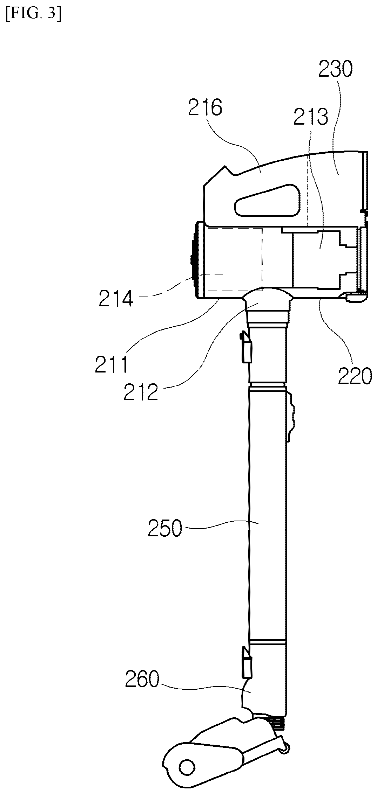

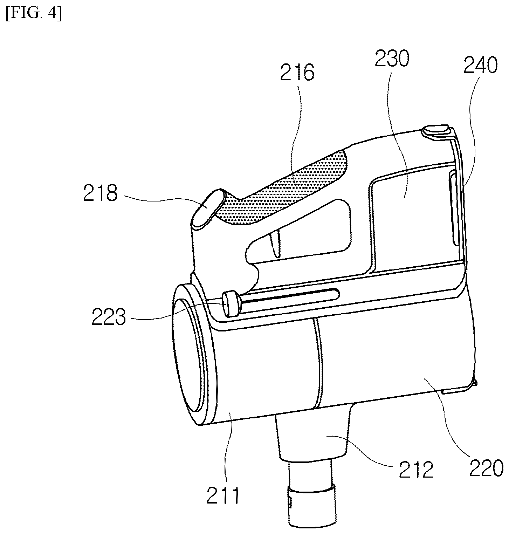

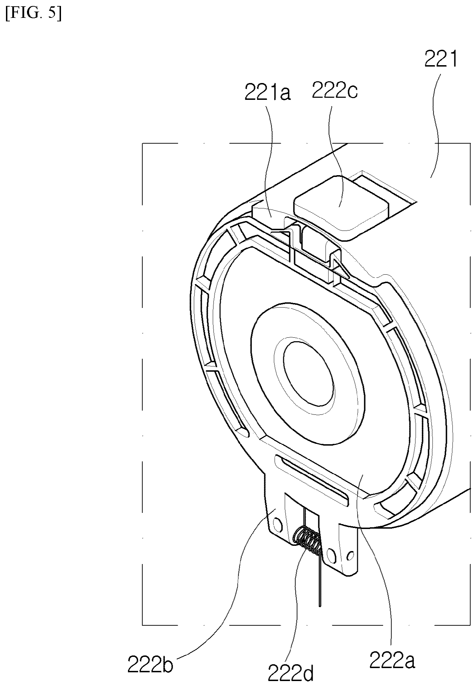

Meanwhile, FIGS. 3 and 4 are views for describing the cleaner of the cleaner system according to the embodiment of the present invention, and FIG. 5 is a view for describing a lower surface of a dust bin of the cleaner according to the embodiment of the present invention.

First, a structure of the cleaner 200 will be described with reference to FIGS. 1 to 5.

The cleaner 200 may be a cleaner which is manually manipulated by a user. For example, the cleaner 200 may be a hand cleaner or a stick cleaner.

The cleaner 200 may be caught on the cleaner station 100. The cleaner 200 may be supported by the cleaner station 100. The cleaner 200 may be coupled to the cleaner station 100.

Meanwhile, in one embodiment of the present invention, a direction of the cleaner 200 can be defined based on when bottom surfaces (lower surfaces) of the dust bin 220 and a battery housing 230 are placed on the ground.

In this case, the front may be a direction in which a suction unit 212 is disposed with respect to a suction motor 214, and the rear may be a direction in which a handle 216 is disposed with respect to the suction motor 214. In addition, a direction disposed at the right may be referred to as a right side, and a direction disposed at the left may be referred to as a left side based on when viewing the suction unit 212 from the suction motor 214. In addition, in one embodiment of the present invention, top and bottom can be defined in a direction perpendicular to the ground with respect to when the bottom surfaces (lower surfaces) of the dust bin 220 and the battery housing 230 are placed on the ground.

The cleaner 200 may include a main body 210. The main body 210 may include a main body housing 211, the suction unit 212, a dust separator 213, the suction motor 214, an air discharging cover 215, the handle 216, and a manipulation unit 218.

The main body housing 211 may form an exterior of the cleaner 200. The main body housing 211 may provide a space in which the suction motor 214 and a filter (not shown) may be accommodated. The main body housing 211 may be formed in a shape similar to a cylinder.

The suction unit 212 may protrude outward from the main body housing 211. For example, the suction unit 212 may be formed in a cylindrical shape with an open interior. The suction unit 212 may be coupled to an extension pipe 250. The suction unit 212 may provide a flow path along which air including dust may flow.

Meanwhile, in the present embodiment, a virtual line passing through the suction unit 212 configured in a cylindrical shape may be formed.

The dust separator 213 may communicate with the suction unit 212. The dust separator 213 may separate dust suctioned therein through the suction unit 212. An internal space of the dust separator 213 may communicate with an internal space of the dust bin 220.

For example, the dust separator 213 may have at least one cyclone unit capable of separating dust by a cyclonic flow. In addition, the internal space of the dust separator 213 may be connected to the suction unit 212. Accordingly, the air and dust suctioned through the suction unit 212 spirally flow along an inner surface of the dust separator 213. Accordingly, the cyclonic flow may occur in the internal space of the dust separator 213.

The dust separator 213 is a component which communicates with the suction unit 212 and adopts the principle of a dust collection unit using a centrifugal force to separate dust suctioned into the main body 210 through the suction unit 212.

The dust separator 213 may further include a secondary cyclone which re-separates dust from the air discharged from the cyclone. In this case, the secondary cyclone may be positioned inside the cyclone to minimize a size of the dust separator. The secondary cyclone may include a plurality of cyclone bodies disposed in parallel. The air discharged from the cyclone may be partitioned into the plurality of cyclone bodies and may pass through the partitioned cyclone bodies.

In this case, an axis of the cyclonic flow of the secondary cyclone may also extend vertically, and an axis of the cyclonic flow of the cyclone and the axis of the cyclonic flow of the secondary cyclone may be coaxial vertically and may be collectively referred to as an axis of the cyclonic flow of the dust separator 213.

The suction motor 214 may generate a suction force of suctioning air. The suction motor 214 may be accommodated in the main body housing 211. The suction motor 214 may generate a suction force by rotation. For example, the suction motor 214 may be provided similar to a cylindrical shape.

Meanwhile, in the present embodiment, a virtual suction motor axial line extending a rotational axis of the suction motor 214 may be formed.

The air discharging cover 215 may be disposed at one side of the main body housing 211 in an axial direction. The air discharging cover 215 may accommodate a filter for filtering air. For example, the air discharging cover 215 may accommodate a HEPA filter.

An air outlet 215a through which the air suctioned by the suction force of the suction motor 214 is discharged may be formed on the air discharge cover 215.

A flow guide may be disposed on the air discharging cover 215. The flow guide may guide a flow of the air discharged through the air outlet 215a.

The handle 216 may be gripped by a user. The handle 216 may be disposed behind the suction motor 214. For example, the handle 216 may be formed in a shape similar to a cylindrical shape. Alternatively, the handle 216 may be formed in a curved cylindrical shape. The handle 216 may be disposed at a predetermined angle with the main body housing 211, the suction motor 214, or the dust separator 213.

The handle 216 may include a grip portion formed in the form of a pillar to allow the user to grip the same, a first extension connected to one end portion in a longitudinal direction (axial direction) of the grip portion and formed to extend toward the suction motor 214, and a second extension connected to the other end portion in the direction (axial direction) of the grip portion and formed to extend toward the dust bin 220.

Meanwhile, in the present embodiment, a virtual grip portion through line formed to extend in a longitudinal direction of the grip portion (axial direction of a pillar) and passing through the grip portion may be formed.

For example, the grip portion through line may be a virtual line formed inside the cylindrical handle 216 and may be a virtual line formed parallel to at least a portion of an outer surface (outer perimetric surface) of the grip portion.

An upper surface of the handle 216 may form a partial exterior of an upper surface of the cleaner 200. Accordingly, when the user grips the handle 216, one component of the cleaner 200 can be prevented from being in contact with an arm of the user.

The first extension may extend from the grip portion toward the main body housing 211 or the suction motor 214. At least a portion of the first extension may extend in a horizontal direction.

The second extension may extend from the grip portion toward the dust bin 220. At least a portion of the second extension may extend in the horizontal direction.

The manipulation unit 218 may be disposed on the handle 216. The manipulation unit 218 may be disposed on an inclined surface formed in an upper area of the handle 216. The user may input an operation or stop command of the cleaner 200 through the manipulation unit 218.

The cleaner 200 may include the dust bin 220. The dust bin 220 may communicate with the dust separator 213. The dust bin 220 may store the dust separated by the dust separator 213.

The dust bin 220 may include a dust bin main body 221, a discharging cover 222, a dust bin compression lever 223, and a compression member (not shown).

The dust bin main body 221 may provide a space in which the dust separated by the dust separator 213 may be stored. For example, the dust bin main body 221 may be formed in a shape similar to a cylindrical shape.

Meanwhile, in the present embodiment, a virtual dust bin center axis, which penetrates the inside (internal space) of the dust bin main body 221 and formed to extend in the longitudinal direction (which is an axial direction in the cylindrical dust bin main body 221) of the dust bin main body 221, may be formed. In this case, the dust bin center axis may be formed to pass through the center of the cross section when viewed in a cross-sectional direction perpendicular to the longitudinal direction of the dust bin main body 221.

A portion of a lower surface (bottom surface) of the dust bin main body 221 may be opened. In addition, a lower surface extension 221a may be formed on the lower surface (bottom surface) of the dust bin main body 221. The lower surface extension 221a may be formed to block a portion of the lower surface of the dust bin main body 221.

The dust bin 220 may include the discharging cover 222. The discharging cover 222 may be disposed on the lower surface of the dust bin 220.

The discharging cover 222 may be provided to open and close one end portion of the dust bin main body 221 in the longitudinal direction. Specifically, the discharging cover 222 may selectively open and close a downward open lower portion of the dust bin 220.

The discharging cover 222 may include a cover main body 222a and a hinge unit 222b. The cover main body 222a may be formed to block a portion of the lower surface of the dust bin main body 221. The cover main body 222a may rotate downward from the hinge unit 222b. The hinge unit 222b may be disposed adjacent to the battery housing 230. The hinge unit 222b may have a torsion spring 222d. Accordingly, when the discharging cover 222 is separated from the dust bin main body 221, the cover main body 222a may be supported while rotated about the hinge unit 222b at a predetermined angle or more in the dust bin main body 221 by an elastic force of the torsion spring 222d.

The discharging cover 222 may be coupled to the dust bin 220 through hook coupling. Meanwhile, the discharging cover 222 may be separated from the dust bin 220 through a coupling lever 222c. The coupling lever 222c may be disposed at the front of the dust bin. Specifically, the coupling lever 222c may be disposed on a front outer surface of the dust bin 220. When an external force is applied, the coupling lever 222c may elastically deform a hook formed to extend from the cover main body 222a to release the hook coupling between the cover main body 222a and the dust bin main body 221.

When the discharging cover 222 is closed, the lower surface of the dust bin 220 may be blocked (sealed) by the discharging cover 222 and the lower surface extension 221a.

The dust bin 220 may include the dust bin compression lever 223 (see FIG. 4). The dust bin compression lever 223 may be disposed outside the dust bin 220 or the dust separator 213. The dust bin compression lever 223 may be disposed to move upward and downward outside the dust bin 220 or the dust separator 213. The dust bin compression lever 223 may be connected to a compression member (not shown). When the dust bin compression lever 223 is moved downward by an external force, the compression member (not shown) may also move downward. Accordingly, user convenience can be provided. The compression member (not shown) and the dust bin compression lever 223 may be returned to original positions by an elastic member (not shown). Specifically, when the external force applied to the dust bin compression lever 223 is removed, the elastic member may move upward the dust bin compression lever 223 and the compression member (not shown).

The compression member (not shown) may be disposed inside the dust bin main body 221. The compression member may move in an internal space of the dust bin main body 221. Specifically, the compression member may move upward and downward in the dust bin main body 221. Accordingly, the compression member may compress the dust in the dust bin main body 221 downward. In addition, when the discharging cover 222 is separated from the dust bin main body 221 and the lower portion of the dust bin 220 is opened, the compression member may move from the upper portion to the lower portion of the dust bin 220 to remove foreign substances such as the remaining dust and the like of the dust bin 220. Accordingly, it is possible to increase the suction force of the cleaner by preventing the remaining dust from remaining in the dust bin 220. In addition, bad odors generated by the residue can be removed by preventing the remaining dust from remaining in the dust bin 220.

The cleaner 200 may include the battery housing 230. A battery 240 may be accommodated in the battery housing 230. The battery housing 230 may be disposed under the handle 216. For example, the battery housing 230 may have a hexahedral shape with an open lower portion. A rear surface of the battery housing 230 may be connected to the handle 216.

The battery housing 230 may include an accommodation portion which is opened downward. The battery 240 may be detachably attached through the accommodation portion of the battery housing 230.

The cleaner 200 may include the battery 240.

For example, the battery 240 may be detachably coupled to the cleaner 200. The battery 240 may be detachably coupled to the battery housing 230. For example, the battery 240 may be inserted into the battery housing 230 from the bottom of the battery housing 230. With this configuration, it is possible to improve the portability of the cleaner 200.

Alternatively, the battery 240 may be provided integrally inside the battery housing 230. In this case, a lower surface of the battery 240 is not exposed to the outside.

The battery 240 may supply power to the suction motor 214 of the cleaner 200. The battery 240 may be disposed under the handle 216. The battery 240 may be disposed behind the dust bin 220.

According to the embodiment, when the battery 240 is coupled to the battery housing 230, the lower surface of the battery 240 may be exposed to the outside. Since the battery 240 may be disposed on the floor when the cleaner 200 is disposed on the floor, the battery 240 may be immediately separated from the battery housing 230. In addition, since the lower surface of the battery 240 is exposed to the outside and comes into direct contact with external air of the battery 240, it is possible to improve cooling performance of the battery 240.

Meanwhile, when the battery 240 is integrally fixed to the battery housing 230, a structure for detachably attaching the battery 240 to the battery housing 230 can be smaller, thereby reducing the overall size of the cleaner 200 and achieving lightweight.

The cleaner 200 may include the extension pipe 250. The extension pipe 250 may communicate with a cleaning module 260. The extension pipe 250 may communicate with the main body 210. The extension pipe 250 may communicate with the suction unit 212 of the main body 210. The extension pipe 250 may be formed in a long cylindrical shape.

The main body 210 may be connected to the extension pipe 250. The main body 210 may be connected to the cleaning module 260 through the extension pipe 250. The main body 210 may generate the suction force through the suction motor 214 and provide the suction force to the cleaning module 260 through the extension pipe 250. External dust may flow into the main body 210 through the cleaning module 260 and the extension pipe 250.

The cleaner 200 may include the cleaning module 260. The cleaning module 260 may communicate with the extension pipe 250. Accordingly, external air may pass through the cleaning module 260 and the extension pipe 250 and may be introduced into the main body 210 of the cleaner 200 by the suction force generated from the main body 210 of the cleaner 200.

Dust in the dust bin 220 of the cleaner 200 may be collected in a dust collection unit 170 of the cleaner station 100 by gravity and the suction force of a dust collection motor 191. Accordingly, since the dust of the dust bin can be removed without the user's separate manipulation, user convenience can be provided. In addition, it is possible to eliminate the inconvenience of the user having to empty the dust bin every time. In addition, it is possible to prevent dust from flying when the user empties the dust bin.

The cleaner 200 may be coupled to a side surface of a housing 110. Specifically, the main body 210 of the cleaner 200 may be caught on a coupling unit 120. More specifically, the dust bin 220 and the battery housing 230 of the cleaner 200 may be coupled to a coupling surface 121, an outer circumferential surface of the dust bin main body 221 may be coupled to a dust bin guide surface 122, and the suction unit 212 may be coupled to a suction unit guide surface 126 of the coupling unit 120. In this case, a center axis of the dust bin 220 may be disposed in a direction parallel to the ground, and the extension pipe 250 may be disposed in a direction perpendicular to the ground.

The cleaner station 100 of the present invention will be described with reference to FIGS. 1 and 2 as follows.

The cleaner 200 may be disposed in the cleaner station 100. The cleaner 200 may be coupled to a side surface of the cleaner station 100. Specifically, the main body of the cleaner 200 may be coupled to the side surface of the cleaner station 100. The cleaner station 100 may remove dust of a dust bin 220 of the cleaner 200.

The cleaner station 100 may include the housing 110. The housing 110 may form the exterior of the cleaner station 100. Specifically, the housing 110 may be formed in a pillar shape including at least one outer wall surface. For example, the housing 110 may be formed in a shape similar to a quadrangular pillar.

The housing 110 may have a space in which the dust collection unit 170 for storing dust therein and a dust suction module 190 for generating a flow force to collect dust into the dust collection unit 170 may be accommodated.

The housing 110 may include a bottom surface 111, an outer wall surface 112, and an upper surface 113.

The bottom surface 111 may support a lower side of the dust suction module 190 in a direction of gravity. That is, the bottom surface 111 may support the lower side of the dust collection motor 191 of the dust suction module 190.

In this case, the bottom surface 111 may be disposed toward the ground. The bottom surface 111 may be not only disposed parallel to the ground, but also disposed to be inclined at a predetermined angle with the ground. With this configuration, there is an advantage that the dust collection motor 191 can be stably supported and the overall weight can be balanced even when the cleaner 200 is coupled.

Meanwhile, according to an embodiment, the bottom surface 111 may further include a ground support 111a which increases an area in contact with the ground to prevent the cleaner station 100 from falling down and maintain balance. For example, the ground support portion may be in the form of a plate extending from the bottom surface 111, and one or more frames may be formed to protrude and extend from the bottom surface 111 in the direction of the ground.

The outer wall surface 112 may be a surface formed in the direction of gravity and may be a surface connected to the bottom surface 111. For example, the outer wall surface 112 may be a surface connected perpendicular to the bottom surface 111. In another embodiment, the outer wall surface 112 may be disposed to be inclined at a predetermined angle with the bottom surface 111.

The outer wall surface 112 may include at least one surface. For example, the outer wall surface 112 may include a first outer wall surface 112a, a second outer wall surface 112b, a third outer wall surface 112c, and a fourth outer wall surface 112d.

In this case, in the present embodiment, the first outer wall surface 112a may be disposed on a front surface of the cleaner station 100. Here, the front surface may be a surface on which the cleaner 200 is exposed in a state in which the cleaner 200 is coupled to the cleaner station 100. Accordingly, the first outer wall surface 112a may form an exterior of the front surface of the cleaner station 100.

Meanwhile, for understanding of the present embodiment, directions are defined as follows. In the present embodiment, directions can be defined in a state in which the cleaner 200 is mounted on the cleaner station 100.

When the cleaner 200 is caught on the cleaner station 100, a direction in which the cleaner 200 is exposed externally from the cleaner station 100 may be referred to as the front.

From another perspective, when the cleaner 200 is caught on the cleaner station 100, a direction in which the suction motor 214 of the cleaner 200 is disposed may be referred to as the front. In addition, a direction opposite to the direction in which the suction motor 214 is disposed in the cleaner station 100 may be referred to as the rear.

In addition, a surface in a direction facing the front surface based on the internal space of the housing 110 may be referred to as the rear surface of the cleaner station 100. Accordingly, the rear surface may refer to a direction in which the second outer wall surface 112b is formed.

In addition, when viewing the front surface with respect to the internal space of the housing 110, a surface of the left may be referred to as a left surface, and a surface of the right may be referred to as a right surface. Accordingly, the left surface may refer to a direction in which the third outer wall surface 112c is formed, and the right surface may refer to the direction in which the fourth outer wall surface 112d is formed.

The first outer wall surface 112a may be formed not only in a flat surface shape, but also entirely in a curved shape, and a portion thereof is formed to have a curved surface.

The first outer wall surface 112a may have the appearance corresponding to the shape of the cleaner 200. Specifically, the coupling unit 120 may be disposed on the first outer wall surface 112a. With this configuration, the cleaner 200 may be coupled to the cleaner station 100 and supported by the cleaner station 100. A specific configuration of the coupling unit 120 will be described below.

Meanwhile, a structure in which various types of cleaning modules 260 used in the cleaner 200 are caught may be added to the first outer wall surface 112a.

In the present embodiment, the second outer wall surface 112b may be a surface facing the first outer wall surface 112a. That is, the second outer wall surface 112b may be disposed on the rear surface of the cleaner station 100. Here, the rear surface may be a surface facing the surface to which the cleaner 200 is coupled. Accordingly, the second outer wall surface 112b may form an exterior of the rear surface of the cleaner station 100.

For example, the second outer wall surface 112b may be formed in a flat surface shape. With this configuration, the second outer wall surface 112b can bring the cleaner station 100 into close contact with a wall in a room and stably support the cleaner station 100.

As another example, a structure for mounting various types of cleaning modules 260 used in the cleaner 200 may be added to the second outer wall surface 112b.

In the present embodiment, the third outer wall surface 112c and the fourth outer wall surface 112d may be surfaces that connect the first outer wall surface 112a to the second outer wall surface 112b. In this case, the third outer wall surface 112c may be disposed on the left surface of the cleaner station 100, and the fourth outer wall surface 112d may be disposed on the right surface of the cleaner station 100. Alternatively, the third outer wall surface 112c may be disposed on the right surface of the cleaner station 100, and the fourth outer wall surface 112d may be disposed on the left surface of the cleaner station 100.

The third outer wall surface 112c or the fourth outer wall surface 112d may be formed not only in a flat surface shape, but also entirely in a curved shape, and a portion thereof may be formed to include a curved surface.

Meanwhile, a structure in which various types of cleaning modules 260 used in the cleaner 200 are caught may be added to the third outer wall surface 112c or the fourth outer wall surface 112d.

The upper surface 113 may form an exterior of the upper side of the cleaner station. That is, the upper surface 113 may be a surface that is disposed on an uppermost side of the cleaner station in the direction of gravity and exposed to the outside in the cleaner station.

For reference, in the present embodiment, an upper side and a lower side may be an upper side and a lower side, respectively, in the direction of gravity (in a direction perpendicular to the ground) in a state in which the cleaner station 100 is installed on the ground.

In this case, the upper surface 113 may be disposed not only parallel to the ground, but also to be inclined at a predetermined angle with the ground.

A display unit 410 may be disposed on the upper surface 113. For example, the display unit 410 may display the state of the cleaner station 100 and the state of the cleaner 200 and also display information such as a cleaning progress, a map of a cleaning zone, and the like.

Meanwhile, according to an embodiment, the upper surface 113 may be provided to be separated from the outer wall surface 112. In this case, when the upper surface 113 is separated, a battery separated from the cleaner 200 may be accommodated in an internal space surrounded by the outer wall surface 112 and provided with a terminal (not shown) for charging the separated battery.

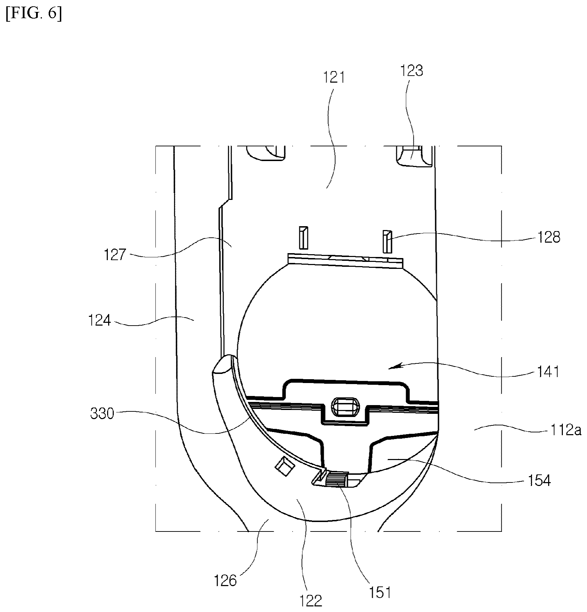

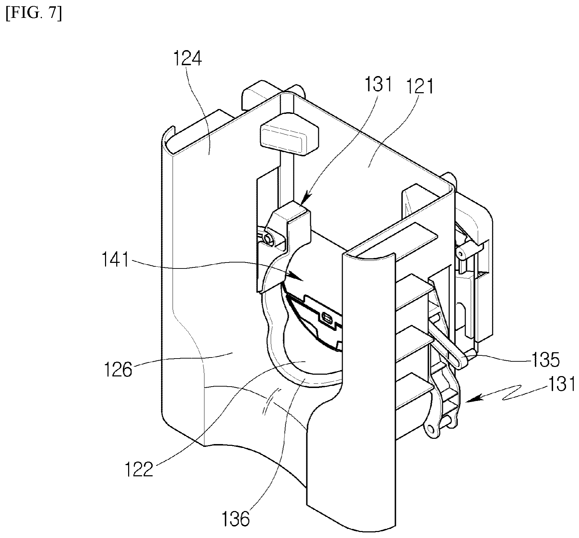

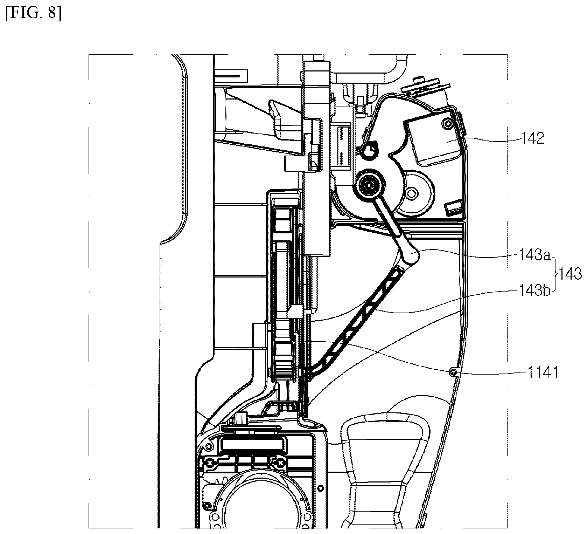

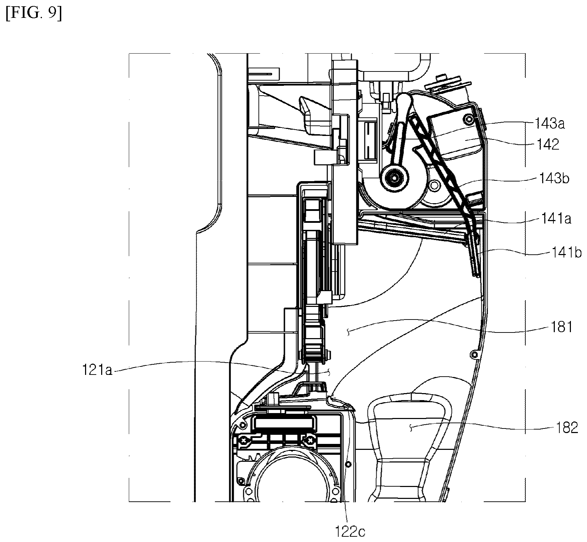

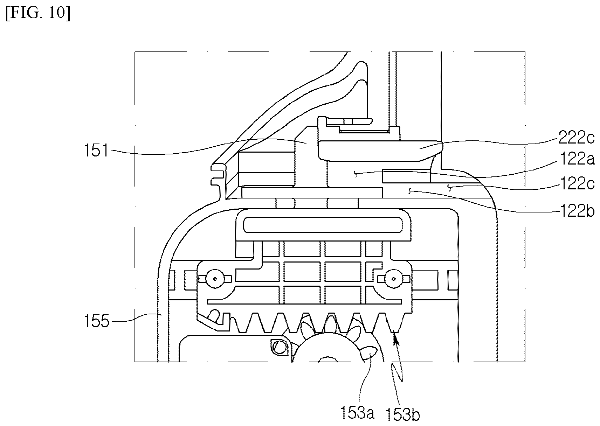

FIG. 6 is a view for describing a coupling unit in the cleaner station according to the embodiment of the present invention, FIG. 7 is an exploded perspective view for describing a fixing unit in the cleaner station according to the embodiment of the present invention, FIGS. 8 and 9 are views for describing a relationship between the cleaner and a door unit in the cleaner station according to the embodiment of the present invention, and FIG. 10 is a view for describing a relationship between the cleaner and a cover open unit in the cleaner station according to the embodiment of the present invention.

The coupling unit 120 of the cleaner station 100 of the present invention will be described with reference to FIGS. 2 and 6 as follows.

The cleaner station 100 may include the coupling unit 120 to which the cleaner 200 is coupled. Specifically, the coupling unit 120 may be disposed on the first outer wall surface 112a, and the main body 210, the dust bin 220, and the battery housing 230 of the cleaner 200 may be coupled.

The coupling unit 120 may include the coupling surface 121. The coupling surface 121 may be disposed on a side surface of the housing 110. For example, the coupling surface 121 may be a surface formed in a groove shape that is concave from the first outer wall surface 112a toward the interior of the cleaner station 100. That is, the coupling surface 121 may be a surface formed by forming a step with the first outer wall surface 112a.

The cleaner 200 may be coupled to the coupling surface 121. As an example, the coupling surface 121 may be in contact with the lower surfaces of the dust bin 220 and the battery housing 230 of the cleaner 200. Here, the lower surface may be a surface toward the ground when the user uses the cleaner 200 or arranges the cleaner 200 on the ground.

For example, an angle formed by the coupling surface 121 and the ground may be a right angle. Accordingly, when the cleaner 200 is coupled to the coupling surface 121, it is possible to minimize a space of the cleaner station 100.

As another example, the coupling surface 121 may be disposed to be inclined at a predetermined angle with the ground. Accordingly, when the cleaner 200 is coupled to the coupling surface 121, the cleaner station 100 can be stably supported.

A dust through hole 121a may be formed in the coupling unit 120 so that external air of the housing 110 may flow therein. Specifically, the dust through hole 121a may be formed in the coupling surface 121 of the coupling unit 120 so that external air of the housing 110 may flow into the housing 110. A dust through hole 121a may be formed in the coupling surface 121 so that external air of the housing 110 may flow into the housing 110. The dust through hole 121a may be formed in a hole shape to correspond to the shape of the dust bin 220 so that the dust of the dust bin 220 flows into the dust collection unit 170. The dust through hole 121a may be formed to correspond to the shape of the discharging cover 222 of the dust bin 220. The dust through hole 121a may be formed to be connected to a suction flow path unit 180 to be described below.

The coupling unit 120 may include the dust bin guide surface 122. The dust bin guide surface 122 may be disposed on the first outer wall surface 112a. The dust bin guide surface 122 may be connected to the first outer wall surface 112a. In addition, the dust bin guide surface 122 may be connected to the coupling surface 121.

The dust bin guide surface 122 may be formed in a shape corresponding to the outer surface of the dust bin 220. The front outer surface of the dust bin 220 may be coupled to the dust bin guide surface 122. Accordingly, the convenience of coupling the cleaner 200 to the coupling surface 121 can be provided.

Meanwhile, a protrusion movement hole 122a may be formed in the dust bin guide surface 122, and a push protrusion 151 described below may move linearly along the protrusion movement hole 122a. In addition, a gear box 155 for accommodating a gear and the like of a cover open unit 150 to be described below may be provided under the dust bin guide surface 122 in the direction of gravity. In this case, a guide space 122b in which the push protrusion 151 may move may be formed between a lower surface of the dust bin guide surface 122 and an upper surface of the gear box 155. In addition, the guide space 122b may be connected to the suction flow path unit 180 through a bypass hole 122c. That is, the protrusion movement hole 122a, the guide space 122b, the bypass hole 122c, and the suction flow path unit 180 may form one bypass flow path (see FIG. 10). With this configuration, when the dust collection motor 191 is operated in a state in which the dust bin 220 is coupled to the coupling unit 120, there is an advantage in that dust, etc. remaining on the dust bin 220 and the dust bin guide surface 122 may be suctioned through the bypass flow path.

The coupling unit 120 may include a guide protrusion 123. The guide protrusion 123 may be disposed on the coupling surface 121. The guide protrusion 123 may protrude upward from the coupling surface 121. Two guide protrusions 123 may be disposed to be spaced apart from each other. A distance between the two guide protrusions 123 spaced apart from each other may correspond to a width of the battery housing 230 of the cleaner 200. Accordingly, the convenience of coupling the cleaner 200 to the coupling surface 121 can be provided.

The coupling unit 120 may include sidewalls 124. The sidewalls 124 may be wall surfaces disposed on both side surfaces of the coupling surface 121 and vertically connected to the coupling surface 121. The sidewall 124 may be connected to the first outer wall surface 112a. In addition, the sidewall 124 may form a surface connected to the dust bin guide surface 122. Accordingly, the cleaner 200 can be stably accommodated.

The coupling unit 120 may include a coupling sensor 125. The coupling sensor 125 may detect whether the cleaner 200 is coupled to the coupling unit 120.

The coupling sensor 125 may include a contact sensor. For example, the coupling sensor 125 may include a micro switch. In this case, the coupling sensor 125 may be disposed on the guide protrusion 123. Accordingly, when the battery housing 230 or the battery 240 of the cleaner 200 is coupled between the pair of guide protrusions 123, the cleaner 200 may come into contact with the coupling sensor 125, and the coupling sensor 125 may detect that the cleaner 200 has been coupled.

Meanwhile, the coupling sensor 125 may include a non-contact sensor. For example, the coupling sensor 125 may include an infrared (IR) sensor. In this case, the coupling sensor 125 may be disposed on the sidewall 124. Accordingly, when the dust bin 220 or the main body 210 of the cleaner 200 passes through the sidewall 124 and reaches the coupling surface 121, the coupling sensor 125 may detect the presence of the dust bin 220 or the main body 210.

The coupling sensor 125 may face the dust bin 220 or the battery housing 230 of the cleaner 200.

The coupling sensor 125 may be a member of determining whether power is applied to the battery 240 of the cleaner 200 and whether the cleaner 200 has been coupled.

The coupling unit 120 may include a suction unit guide surface 126. The suction unit guide surface 126 may be disposed on the first outer wall surface 112a. The suction unit guide surface 126 may be connected to the dust bin guide surface 122. The suction unit 212 may be coupled to the suction unit guide surface 126. A shape of the suction unit guide surface 126 may be formed in a shape corresponding to the shape of the suction unit 212.

The coupling unit 120 may further include a fixing member entrance hole 127. The fixing member entrance hole 127 may be formed in the form of a long hole along the sidewall 124 to allow a fixing member 131 to enter and exit the same.

With this configuration, when the user couples the cleaner 200 to the coupling unit 120 of the cleaner station 100, the main body 210 of the cleaner 200 can be stably disposed on the coupling unit 120 by the dust bin guide surface 122, the guide protrusion 123, and the suction unit guide surface 126. Accordingly, the convenience of coupling the dust bin 220 and the battery housing 230 of the cleaner 200 to the coupling surface 121 can be provided.

A fixing unit 130 according to the present invention will be described with reference to FIGS. 2 and 7 as follows.

The cleaner station 100 according to the present invention may include the fixing unit 130. The fixing unit 130 may be disposed on the sidewall 124. In addition, at least a portion of the fixing unit 130 may be disposed on a rear surface of the coupling surface 121. The fixing unit 130 may fix the cleaner 200 coupled to the coupling surface 121. Specifically, the fixing unit 130 may fix the dust bin 220 and the battery housing 230 of the cleaner 200 coupled to the coupling surface 121.

The fixing unit 130 may include a fixing member 131 for fixing the dust bin 220 and the battery housing 230 of the cleaner 200, and a fixing unit motor 133 for driving the fixing member 131. In addition, the fixing unit 130 may further include a fixing unit link 135 for transmitting power of the fixing unit motor 133 to the fixing member 131.

The fixing member 131 may be disposed on the sidewall 124 of the coupling unit 120 and provided to reciprocate on the sidewall 124 to fix the dust bin 220. Specifically, the fixing member 131 may be accommodated inside the fixing member entrance hole 127.

The fixing member 131 may be disposed on each of both sides of the coupling unit 120. For example, a pair of two fixing members 131 may be disposed symmetrically centered on the coupling surface 121.

The fixing unit motor 133 may provide power for moving the fixing member 131.

The fixing unit link 135 may convert a rotational force of the fixing unit motor 133 into reciprocating movement of the fixing unit member 131.

When coupled to the cleaner 200, a fixing sealer 136 may be disposed on the dust bin guide surface 122 to airtighten the dust bin 220. With this configuration, when the dust bin 220 of the cleaner 200 is coupled, the fixing sealer 136 may be pressed by the weight of the cleaner 200, and the dust bin 220 and the dust bin guide surface 122 may be sealed.

The fixing sealer 136 may be disposed on a virtual extension line of the fixing member 131. With this configuration, when the fixing unit motor 133 is operated so that the fixing member 131 presses the dust bin 220, a perimeter of the dust bin 220 at the same height may be sealed.

According to an embodiment, the fixing sealer 136 may be disposed on the dust bin guide surface 122 in a bent line shape corresponding to the arrangement of a cover open unit 150 to be described below.

Accordingly, when the main body 210 of the cleaner 200 is disposed on the coupling unit 120, the fixing unit 130 may fix the main body 210 of the cleaner 200. Specifically, when the coupling sensor 125 detects that the main body 210 of the cleaner 200 is coupled to the coupling unit 120 of the cleaner station 100, the fixing unit motor 133 may fix the main body 210 of the cleaner 200 by moving the fixing member 131.

Accordingly, it is possible to increase the suction force of the cleaner by preventing the remaining dust from remaining in the dust bin. In addition, bad odors generated by the residue can be removed by preventing the remaining dust from remaining in the dust bin.

A door unit 140 according to the present invention will be described with reference to FIGS. 2, 8, 9, and 11 as follows.

The cleaner station 100 of the present invention may include the door unit 140. The door unit 140 may be formed to open and close the dust through hole 121a.

The door unit 140 may include a door 141, a door motor 142, and a door arm 143.

The door 141 may be hinge-coupled to the coupling surface 121 to open and close the dust through hole 121a. The door 141 may include a door main body 141a.

The door main body 141a may be formed in a shape that may block the dust through hole 121a. For example, the door main body 141a may be formed in a shape similar to a disk shape.

Based on a state in which the door main body 141a blocks the dust through hole 121a, a hinge unit may be disposed above the door body 141a, and an arm coupling portion 141b may be disposed below the door main body 141a.

The door main body 141a may be formed in a shape that may airtighten the dust through hole 121a. For example, an outer surface of the door main body 141a exposed outward from the cleaner station 100 is formed to have a diameter corresponding to a diameter of the dust through hole 121a, and an inner surface of the door main body 141a disposed inside the cleaner station 100 is formed to have a diameter larger than the diameter of the dust through hole 121a. In addition, a step may occur between the outer surface and the inner surface. Meanwhile, at least one reinforcing rib for connecting the hinge unit to the arm coupling portion 141b and reinforcing a support strength of the door main body 141a may be formed to protrude from the inner surface of the door main body 141a.

The hinge unit may be a means for hinge-coupling the door 141 to the coupling surface 121. The hinge unit may be disposed on an upper end portion of the door main body 141a and coupled to the coupling surface 121.

The arm coupling portion 141b may be a means to which the door arm 143 is rotatably coupled. The arm coupling portion 141b may be disposed below the door main body 141a, rotatably coupled to the door main body 141a, and rotatably coupled to the door arm 143.

With this configuration, in a state in which the door 141 closes the dust through hole 121a, when the door arm 143 pulls the door main body 141a, the door main body 141a may move while rotating about the hinge unit inward from the cleaner station 100 to open the dust through hole 121a. Meanwhile, in a state in which the dust through hole 121a is opened, when the door arm 143 pushes the door main body 141a, the door main body 141a may move while rotating about the hinge unit outward from the cleaner station 100 to block the dust through hole 121a.

Meanwhile, in a state in which the cleaner 200 is coupled to the cleaner station 100 and the discharging cover 222 is separated from the dust bin main body 221, the door 141 may be in contact with the discharging cover 222. In addition, as the door 141 rotates, the discharging cover 222 may be rotated in conjunction with the door 141.

The door motor 142 may provide power for rotating the door 141. Specifically, the door motor 142 may rotate the door arm 143 in a forward or reverse direction. Here, the forward direction may be a direction in which the door arm 143 pulls the door 141. Accordingly, when the door arm 143 rotates in the forward direction, the dust through hole 121a may be opened. In addition, the reverse direction may be a direction in which the door arm 143 pushes the door 141. Accordingly, when the door arm 143 rotates in the reverse direction, at least a portion of the dust through hole 121a may be closed. The forward direction may be a direction opposite to the reverse direction.

The door arm 143 may connect the door 141 to the door motor 142 and open and close the door 141 using the power generated by the door motor 142.

For example, the door arm 143 may include a first door arm 143a and a second door arm 143b. One end portion of the first door arm 143a may be coupled to the door motor 142. The first door arm 143a may be rotated by the power of the door motor 142. The other end portion of the first door arm 143a may be rotatably coupled to the second door arm 143b. The first door arm 143a may transmit a force received from the door motor 142 to the second door arm 143b. One end portion of the second door arm 143b may be coupled to the first door arm 143a. The other end portion of the second door arm 143b may be coupled to the door 141. The second door arm 143b may open and close the dust through hole 121a by pushing or pulling the door 141.

The door unit 140 may further include a door opening and closing detection unit 144. The door opening and closing detection unit 144 may be provided inside the housing 110 to detect whether the door 141 is in an open state.

For example, the door opening and closing detection unit 144 may be disposed on each of both end portions of a rotational movement region of the door arm 143. As another example, the door opening and closing detection unit 144 may be disposed on each of both end portions of a movement region of the door 141.

Accordingly, when the door arm 143 moves to a preset door opening position or the door 141 is opened to a predetermined position, the door opening and closing detection unit 144 may detect that the door has been opened. In addition, when the door arm 143 moves to a preset door closed position or the door 141 is opened to a predetermined position, the door opening and closing detection unit 144 may detect that the door has been opened.

The door opening and closing detection unit 144 may include a contact sensor. For example, the door opening and closing detection unit 144 may include a micro-switch.

Meanwhile, the door opening and closing detection unit 144 may include a non-contact sensor. For example, the door opening and closing detection unit 144 may include an IR sensor.

With this configuration, the door unit 140 may selectively open and close at least a portion of the coupling surface 121 so that the outside of the first outer wall surface 112a may be connected to the suction flow path unit 180 and/or the dust collection unit 170.

The door unit 140 may also be opened when the discharging cover 222 of the cleaner 200 is opened. In addition, when the door unit 140 is closed, the discharging cover 222 of the cleaner 200 may also be closed in conjunction with the door unit 140.

When the dust of the dust bin 220 of the cleaner 200 is removed, the door motor 142 may rotate the door 141 to couple the discharging cover 222 to the dust bin main body 221. Specifically, the door motor 142 may rotate the door 141, and the rotating door 141 may push the discharging cover 222 toward the dust bin main body 221.

The cover open unit 150 according to the present disclosure will be described with reference to FIGS. 2, 10, and 11 as follows.

The cleaner station 100 of the present invention may include the cover open unit 150. The cover open unit 150 may be disposed on the coupling unit 120 to open the discharging cover 222 of the cleaner 200.

The cover open unit 150 may include a push protrusion 151, a cover open motor 152, a cover open gear 153, a support plate 154, and a gear box 155.

The push protrusion 151 may move to press the coupling lever 222c when the cleaner 200 is coupled.

The push protrusion 151 may be disposed on the dust bin guide surface 122. Specifically, a protrusion movement hole may be formed in the dust bin guide surface 122, and the push protrusion 151 may be exposed to the outside after passing through the protrusion movement hole.

The push protrusion 151 may be disposed at a position at which it may push the coupling lever 222c when the cleaner 200 is coupled. That is, the coupling lever 222c may be disposed on the protrusion movement hole. In addition, the coupling lever 222c may be disposed on a movement region of the push protrusion 151.

The push protrusion 151 may linearly reciprocate to press the coupling lever 222c. Specifically, the push protrusion 151 may be coupled to the gear box 155 to guide linear movement. The push protrusion 151 may be coupled to the cover open gear 153 and moved together by the movement of the cover open gear 153.

The cover open motor 152 may provide power for moving the push protrusion 151. Specifically, the cover open motor 152 may rotate a motor shaft (not shown) in the forward or reverse direction. Here, the forward direction may be a direction in which the push protrusion 151 presses the coupling lever 222c. In addition, the reverse direction may be a direction in which the push protrusion 151 pressing the coupling lever 222c is returned to an original position. The forward direction may be a direction opposite to the reverse direction.

The cover open gear 153 may be coupled to the cover open motor 152 to move the push protrusion 151 using the power of the cover open motor 152. Specifically, the cover open gear 153 may be accommodated inside the gear box 155. A driving gear 153a of the cover open gear 153 may be coupled to the motor shaft of the cover open motor 152 to receive power. A driven gear 153b of the cover open gear 153 may be coupled to the push protrusion 151 to move the push protrusion 151. As an example, the driven gear 153b may be provided in the form of a rack gear, engaged with the driving gear 153a, and may receive power from the driving gear 153a.

In this case, the discharging cover 222 may have the torsion spring 222d. The discharging cover 222 may be rotated at a predetermined angle or more by an elastic force of the torsion spring 222d and supported at the rotated position. Accordingly, the discharging cover 222 may be opened so that the dust through hole 121a may communicate with the interior of the dust bin 220.

The gear box 155 may be provided inside the housing 110, disposed under the coupling unit 120 in the direction of gravity, and may accommodate the cover open gear 153 therein.

The gear box 155 may have a cover open detection unit 155f. In this case, the cover open detection unit 155f may include a contact sensor. For example, the cover open detection unit 155f may include a micro-switch. Meanwhile, the cover open detection unit 155f may also include a non-contact sensor. For example, the cover open detection unit 155f may include an IR sensor.

At least one cover open detection unit 155f may be disposed on an inner or outer surface of the gear box 155. For example, one cover open detection unit 155f may be disposed on the inner surface of the gear box 155. In this case, the cover open detection unit 155f may detect the push protrusion 151 positioned at an initial position.

As another example, two cover open detection units 155f may be disposed on the outer surface of the gear box 155. In this case, the cover open detection unit 155f may detect the initial position of the push protrusion 151 and the cover open position.

Accordingly, according to the present invention, the dust bin 220 may be opened by the cover open unit 150 without the user separately opening the discharge cover 222 of the cleaner, thereby improving convenience.

In addition, since the discharging cover 222 is opened in a state in which the cleaner 200 is coupled to the cleaner station 100, it is possible to prevent dust from flying.

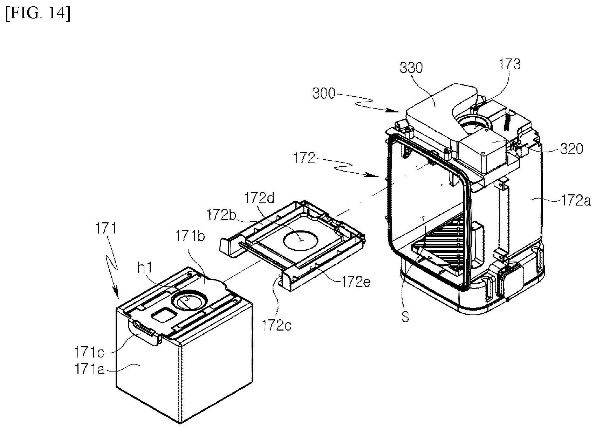

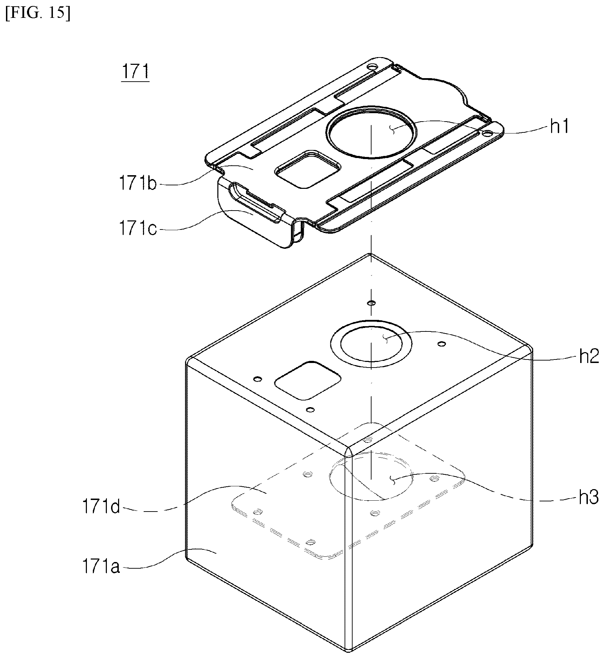

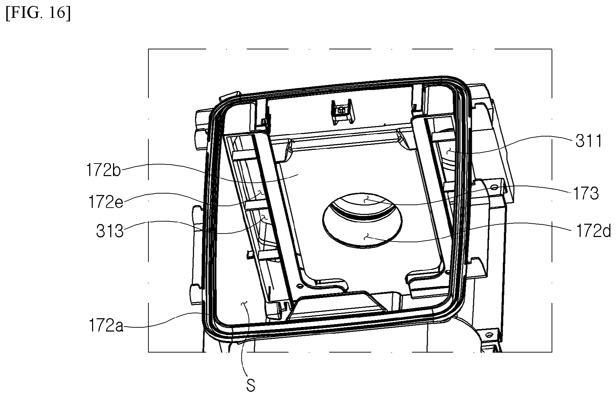

FIG. 14 is an exploded view of main components of a dust collection unit of the cleaner station according to the embodiment of the present invention, FIG. 15 is an exploded view of main components of a dust storage module of the cleaner station according to the embodiment of the present invention, and FIG. 16 is an enlarged view of main components of a dust collection chamber of the cleaner station according to the embodiment of the present invention.

The dust collection unit 170 will be described with reference to FIGS. 2, 11 and 14 to 16 as follows.

The cleaner station 100 may include the dust collection unit 170. The dust collection unit 170 may be disposed inside the housing 110. The dust collection unit 170 may be disposed below the coupling unit 120 in the direction of gravity.

The dust collection unit 170 may collect dust inside the dust bin 220 of the cleaner 200. Specifically, when the dust collection motor 191 is operated in a state in which the cleaner 200 is coupled to the cleaner station 100 and the dust through hole 121a communicates with the suction flow path unit 180, the dust inside the dust bin 220 may be collected in the dust collection unit 170 while flowing along the suction flow path unit 180 by the suction force of the dust collection motor 191.

The dust collection unit 170 may include a dust storage module 171 and a dust collection chamber 172.

The dust storage module 171 is a component for collecting dust inside the dust bin 220 and may be connected to the suction flow path unit 180. That is, the suction flow path unit 180 may have one end connected to the dust through hole 121a and the other end connected to the dust storage module 171. Specifically, the dust storage module 171 may be connected to a second suction flow path 182.

The dust storage module 171 may be detachably coupled to the dust collection chamber 172.

Accordingly, when the dust collection chamber 172 is opened, the dust storage module 171 may be separated from the dust collection chamber 172 and discarded, and a new dust storage module 171 may be coupled to the dust collection chamber 172. That is, the dust storage module 171 may be defined as a consumable component.

The dust storage module 171 may include a dust bag 171a and an upper plate 171b.

The dust bag 171a may be a component for receiving and storing dust suctioned from the dust bin 220.

The dust bag 171a may be provided so that dust is accommodated therein due to an increased volume when a suction force is generated by the dust collection motor 191. To this end, the dust bag 171a may be formed of a material that allows air to pass but blocks foreign substances such as dust. As an example, the dust bag 171a may be formed of a non-woven material and may have a hexahedral shape based on when the volume is increased. However, the dust bag 171a is not limited thereto and may also be formed in other polyhedral or cylindrical shapes.

When an airflow is generated by the suction force of the dust collection motor 191, air including the foreign substances, which flows inside the dust bin 220 of the cleaner 200, moves to the dust bag 171a through the suction flow path unit 180, only the foreign substances remain in the dust bag 171a, and the air may exit the dust bag 171a.

The upper plate 171b may be coupled to an outer upper surface of the dust bag 171a and inserted into a fixed holder 172b of the dust collection chamber 172, which will be described below. The upper plate 171b may be inserted into a mounting groove 172c of the fixed holder 172b in a sliding manner. Insertion of the upper plate 171b may be performed from the front toward the rear of the fixed holder 172b with respect to the fixed holder 172b.

A sliding surface of the upper plate 171b inserted into the mounting groove 172c may be provided in a rectangular shape. In addition, the upper plate 171b may be inserted by sliding from the first outer wall surface 112a of the housing 110 toward the second outer wall surface 112b when inserted into the dust collection chamber 172.

The upper plate 171b may include a dust bag handle 171c.

The dust bag handle 171c may be provided at the front of the dust storage module 171. The dust bag handle 171c may be gripped by the user when the dust storage module 171 is removed from or inserted into the dust collection chamber 172. The sliding surface of the upper plate 171b may be connected to the dust bag handle 171c at a right angle. A user may grip the dust bag handle 171c and push the sliding surface into the mounting groove 172c. The user may grip the dust bag handle 171c and remove the sliding surface from the mounting groove 172c.

The dust storage module 171 may further include a lower plate 171d.

The lower plate 171d may be a component coupled to an inner upper surface of the dust bag 171a. The lower plate 171d may be provided in a rectangular shape. The lower plate 171d may be coupled to the upper plate 171b through a separate fastening means with the dust bag 171a interposed therebetween.

Meanwhile, a dust hole h1 through which air containing dust is introduced may be formed in the upper plate 171b. The dust hole h1 of the upper plate 171b may communicate upward with a dust inlet 173 and a dust hole 172d of the fixed holder 172b, which will be described below.

Like the upper plate 171b, a dust hole h2 through which air containing dust is introduced may also be formed in the upper surface of the dust bag 171a. The dust hole h2 of the dust bag 171a may communicate upward with the dust hole h1 of the upper plate 171b and communicate downward with a dust hole h3 of the lower plate 171d.

Like the upper plate 171b and the dust bag 171a, the dust hole h3 through which air containing dust is introduced may be formed in the lower plate 171d. The dust hole h3 of the lower plate 171d may communicate upward with the dust hole h2 of the dust bag 171a and communicate downward with the inside of the dust bag 171a.

The dust inlet 173 may be connected to the suction flow path unit 180, and consequently, air flowing through the suction flow path unit 180 may pass through the dust hole 172d of the fixed holder 172b and the dust holes h1, h2, and h3 of the dust storage module 171 through the dust inlet 173 and flow into the dust bag 171a.

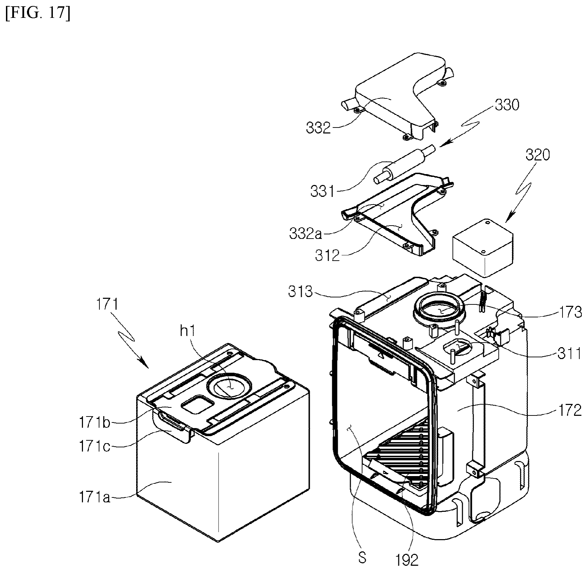

Meanwhile, in the embodiment illustrated in FIGS. 15 and 17, the dust inlet 173 and the dust holes h1, h2, h3, and 172d are each illustrated as having a circular shape, but shapes thereof are not limited to the illustrated embodiment as long as the above functions are maintained.

The cleaner station 100 according to the present invention may further include the dust collection chamber 172.

An internal space S that accommodates the dust storage module 171 may be formed inside the dust collection chamber 172. The dust collection chamber 172 may be provided so that the dust storage module 171 may be detachably attached.

The dust collection chamber 172 may include a chamber housing 172a forming an exterior of the dust collection chamber 172 and the fixed holder 172b that detachably couples the inserted dust storage module 171.

In this case, the internal space S may be a space within the chamber housing 172a in which the dust bag 171a is disposed. For example, the internal space S may be a space with a predetermined distance formed between an outer surface of the dust bag 171a and an inner surface of the chamber housing 172a. As another example, the internal space S may be an internal space of the dust bag 171a. As still another example, the internal space S may be a space formed between the outer surface of the dust bag 171a and the inner surface of the chamber housing 172a and a space including the internal space of the dust bag 171a.

Accordingly, air discharged from a discharge port 313 may flow while circulating in the internal space S. In addition, the internal space S may communicate with the dust suction module 190. Specifically, the internal space S may communicate with an internal space of the dust suction module 190 in which the dust collection motor 191 is disposed.

In the embodiment described herein, an example in which the dust collection chamber 172 is accommodated in a portion of the internal space of the housing 110 and the dust storage module 171 is accommodated within the internal space S of the dust collection chamber 172. That is, the dust collection chamber 172 may be provided separately from the housing 110. However, the embodiment is not limited thereto, and the housing 110 and the dust collection chamber 172 may not be separate components, and the dust collection chamber 172 may form a portion of the housing 110.

Meanwhile, the chamber housing 172a may have a hexahedral shape with one open surface as illustrated in FIG. 14. The dust storage module 171 may be coupled to or separated from the fixed holder 172b through the one open surface.

In this case, a direction in which the open surface of the chamber housing 172a faces may be defined as the front, and one side to which the suction flow path unit 180 is connected may be defined as the top.

The chamber housing 172a may communicate with the suction flow path unit 180 through the dust inlet 173 formed on a top side (upper surface). The dust inlet 173 may be a component for guiding air flowing through the suction flow path unit 180 into the dust bag 171a. That is, one end of the dust inlet 173 may be connected to the suction flow path unit 180, and the other end may be connected to the dust bag 171a.

Accordingly, dust suctioned from the dust bin 220 may move into the dust bag 171a through the suction flow path unit 180 and the dust inlet 173.

The dust collection chamber 172 may include the fixed holder 172b.

The fixed holder 172b may be fixedly disposed on an upper surface of the internal space S of the chamber housing 172a. The fixed holder 172b may be a component for guiding the dust storage module 171 to be inserted into the internal space S of the chamber housing 172a. The fixed holder 172b may be a component for supporting the dust storage module 171 to prevent it from shaking while inserted into the internal space S. In this case, the upper plate 171b of the dust storage module 171 may be slid into the mounting groove 172c provided in the fixed holder 172b for insertion.

The fixed holder 172b may be comprised of a first fixed holder disposed at the left side of the upper surface of the internal space S and a second fixed holder 172b disposed at the right side of the upper surface thereof. The first and second fixed holders may be provided so that the entire upper surfaces are fixed to the upper surface of the internal space S.

That is, the fixed holder 172b is a fixed structure that does not move in any direction and may support the dust storage module 171 to prevent it from shaking within the internal space S.

The fixed holder 172b may include the mounting groove 172c formed to allow the dust storage module 171 to slide. The mounting groove 172c may extend in a front-rear direction so that the dust storage module 171 may be inserted from the front toward the rear. Specifically, the mounting groove of the first fixed holder may have a concave structure in which a right side surface of the first fixed holder is recessed to the left with a predetermined depth and height. The mounting groove of the second fixed holder may have a concave structure in which a left side surface of the second fixed holder is recessed to the right with a predetermined depth and height.

Meanwhile, the fixed holder 172b may be formed with a dust hole 172d through which air containing dust passes. The dust hole 172d formed in the fixed holder 172b may communicate upward with the dust inlet 173 and communicate downward with the dust hole h1 formed in the upper plate 171b. The dust inlet 173 may be connected to the suction flow path unit 180, and consequently, air flowing through the suction flow path unit 180 may pass through the dust hole 172d of the fixed holder 172b and the dust holes h1, h2, and h3 of the dust storage module 171 through the dust inlet 173 and flow into the dust bag 171a.

The fixed holder 172b may be formed with at least one circulation hole 172e that communicates with a suction port 311 and the discharge port 313. Accordingly, air suctioned through the suction port 311 and/or air discharged through the discharge port 313 may pass through the circulation hole 172e without being blocked by the fixed holder 172b and smoothly circulate in the internal space S and a circulation flow path unit 310.

Meanwhile, the dust collection unit 170 may be provided with a temperature sensor 174. The temperature sensor 174 may measure a temperature inside the dust collection unit 170. The temperature information measured by the temperature sensor 174 may be received by a controller 400. The temperature sensor 174 may be disposed in the dust collection unit 170. The temperature sensor 174 may be disposed in the discharge port 313 or in the internal space S. The temperature sensor 174 may measure the temperature of the air discharged from the discharge port 313 and/or the air in the internal space S to calculate the temperature of the air circulating in the circulation flow path unit 310.

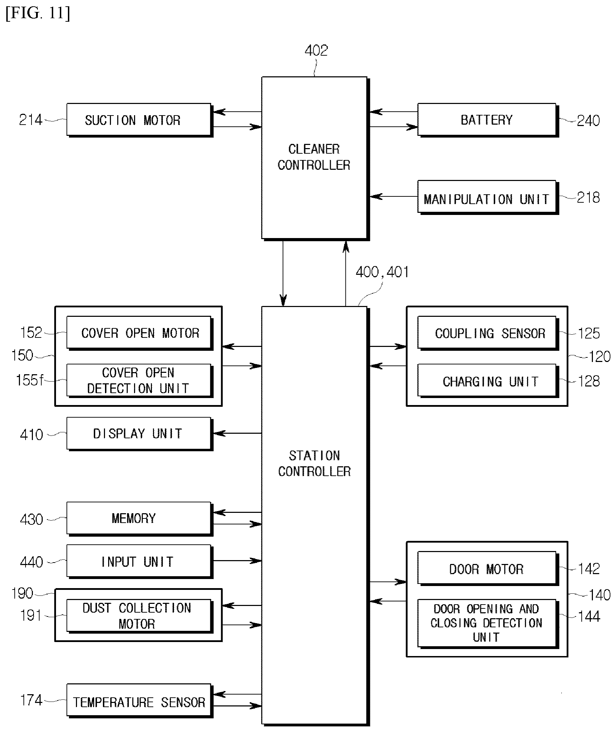

Meanwhile, the suction flow path unit 180 will be described with reference to FIGS. 2 and 11 as follows.

The cleaner station 100 may include the suction flow path unit 180.

The dust inside the dust bin 220 of the cleaner 200 may move to the dust collection unit 170 through the suction flow path unit 180.

The suction flow path unit 180 may connect the dust through hole 121a formed in the coupling surface 121a to the dust collection unit 170. One end of the suction flow path unit 180 may be connected to the dust through hole 121a, and the other end may be connected to the dust collection unit 170. The suction flow path unit 180 may be disposed behind the coupling surface 121. The suction flow path unit 180 may refer to a space between the dust bin 220 of the cleaner 200 and the dust collection unit 170. The suction flow path unit 180 may be a space formed behind the dust through hole 121a and may be a flow path which is formed to be bent downward from the dust through hole 121a and along which dust and air may flow.

Specifically, the suction flow path unit 180 may include a first suction flow path 181 connected to the internal space of the dust bin 220 when the cleaner 200 is coupled to the cleaner station 100 and the dust through hole 121a is opened, and a second suction flow path 182 that connects the first suction flow path 181 to a collection space 1711 of the dust bag 171a.

For example, the first suction flow path 181 may be disposed substantially parallel to an axial line of the suction motor 214 or a virtual through-line passing through the dust bin 220. In this case, the axial line of the suction motor 214 or the through-line of the dust bin 220 may pass through the first suction flow path 181.

In this case, the second suction flow path 182 may be formed at a predetermined angle with the first suction flow path 181. As an example, the first suction flow path 181 and the second suction flow path 182 may be formed at a right angle. With this configuration, it is possible to minimize the overall volume of the cleaner station 100.

Meanwhile, a length of the first suction flow path 181 may be smaller than or equal to a length of the second suction flow path 182. With this configuration, even when the overall flow path for removing dust is bent once, the suction force of the dust collection motor 191 may be transmitted to the internal space of the dust bin 220.

Meanwhile, the dust suction module 190 will be described with reference to FIGS. 2 and 11 as follows.

The cleaner station 100 may include the dust suction module 190. The dust suction module 190 may include the dust collection motor 191, a first filter 192, and a second filter (not shown).

The dust collection motor 191 may be disposed below the dust collection unit 170. The dust collection motor 191 may generate a suction force in the suction flow path unit 180. Accordingly, the dust collection motor 191 may provide a suction force capable of suctioning the dust of the dust bin 220 of the cleaner 200.

The dust collection motor 191 may generate the suction force by rotation. For example, the dust collection motor 191 may be formed in a shape similar to a cylinder.

Meanwhile, in the present embodiment, a virtual dust collection motor axial line C extending a rotational axis of the dust collection motor 191 may be formed.

The first filter 192 may be disposed between the dust collection unit 170 and the dust collection motor 191. The first filter 192 may be a pre-filter. The air that has passed through the first filter 192 may flow into the internal space in which the dust collection motor 191 is accommodated.

The second filter (not shown) may be disposed between the dust collection motor 191 and the outer wall surface 112. The second filter (not shown) may be a HEPA filter.

Meanwhile, the cleaner station 100 may further include a charging unit 128. The charging unit may be disposed on the coupling unit 120. The charging unit 128 may be electrically connected to the cleaner 200 coupled to the coupling unit 120. The charging unit 128 may supply power to the battery of the cleaner 200 coupled to the coupling unit 120.

In addition, the cleaner station 100 may further include a side door (not shown). The side door may be disposed in the housing 110. The side door may selectively expose the dust collection unit 170 to the outside. Accordingly, the user can easily remove the dust collection unit 170 from the cleaner station 100.

In addition, the cleaner station 100 may further include an exhaust port 162. The exhaust port 162 may be formed in the housing 110. For example, the exhaust port 162 may be formed at the lower side of the housing 110 and connected to the dust collection motor 191 through a flow path. Therefore, the air passing through the dust collection motor 191 may be discharged to the outside of the housing 110 through the exhaust port 162.

Meanwhile, an exhaust unit 160 will be described with reference to FIG. 2 as follows.

The cleaner station 100 may further include the exhaust unit 160.

The exhaust unit 160 may guide the air discharged from the dust collection motor 191 to the outside of the housing 110. The exhaust unit 160 may allow the internal space to communicate with an external space of the housing 110.

The exhaust unit 160 may include an exhaust flow path 161. The air discharged from the dust collection motor 191 may flow through the exhaust flow path 161. The exhaust flow path 161 may provide a flow path through which the air discharged from the dust collection motor 191 flows. The air flowing through the exhaust flow path 161 may be discharged to the outside of the housing 110 through the exhaust port 162. Specifically, one end of the exhaust flow path 161 may communicate with the internal space in which the dust collection motor 191 is accommodated of the dust suction module 190, and the other end of the exhaust flow path 161 may communicate with the exhaust port 162. As an example, the exhaust flow path 161 may be a flow path formed in a horizontal direction inside the housing 110, and the exhaust port 162 may allow the inside to communicate with the outside of the housing 110. One end portion of the exhaust flow path161 may communicate with the dust suction module 190, and the other end portion of the exhaust flow path 161 may communicate with the exhaust port 162.

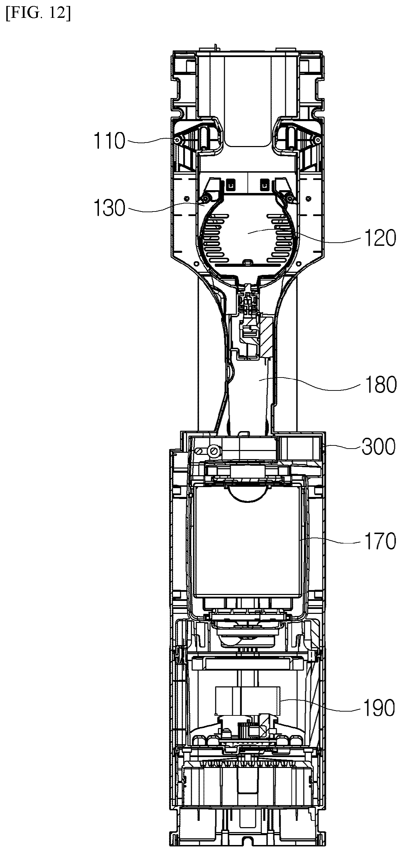

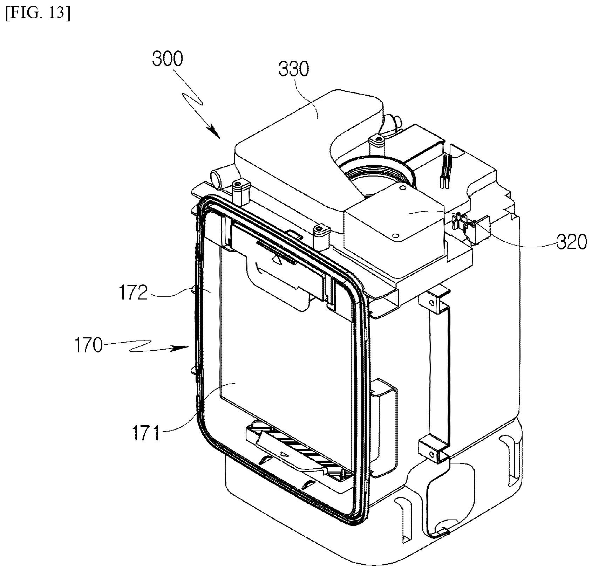

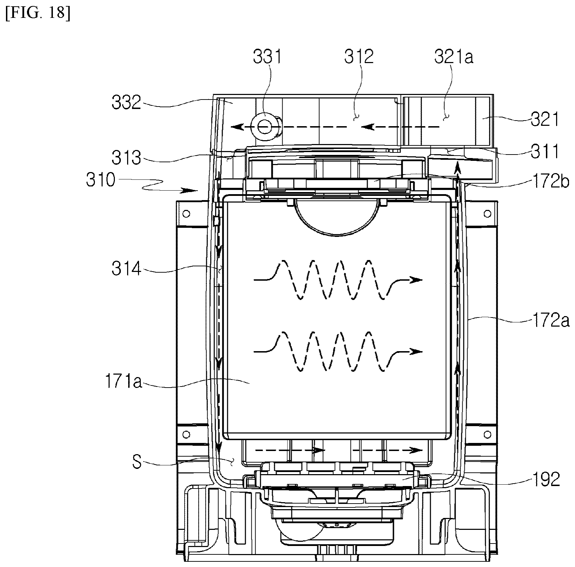

FIG. 12 is a cross-sectional view for describing a specific configuration of the cleaner station according to the embodiment of the present invention, FIG. 13 is a perspective view for describing a heat circulation module of the cleaner station according to the embodiment of the present invention, FIG. 17 is an exploded view for describing the heat circulation module of the cleaner station according to the embodiment of the present invention, and FIG. 18 is a cross-sectional view for describing the heat circulation module of the cleaner station according to the embodiment of the present invention.

Referring to FIGS. 12, 13, 17, and 18, a heat circulation module 300 according to the embodiment of the present invention will be described as follows.

The cleaner station 100 according to the embodiment of the present invention may include the heat circulation module 300.

The heat circulation module 300 may heat air suctioned from the internal space S of the dust collection unit 170 and supply the heated air to the internal space S. Accordingly, air whose temperature has decreased while circulating in the internal space S may be reheated by the heat circulation module 300 and introduced into the internal space S.

The heat circulation module 300 may include the circulation flow path unit 310, a blower 320, and a heating unit 330.

The circulation flow path unit 310 may provide a flow path through which the air in the internal space S may circulate with the outside of the dust collection unit 170. The circulation flow path unit 310 may provide a flow path that guides the air suctioned from the internal space S back into the internal space S.

The circulation flow path unit 310 may include the suction port 311, a first circulation flow path 312, the discharge port 313, and a second circulation flow path 314.

The suction port 311 may serve as an inlet through which the air in the internal space S is suctioned. The air in the internal space S may be suctioned through the suction port 311 and introduced into the first circulation flow path 312.

The suction port 311 may be formed at an inner one side of the dust collection unit 170. Specifically, the suction port 311 may be formed on one side of an upper portion of the chamber housing 172a.

In the embodiment illustrated in FIG. 17, the suction port 311 is illustrated as having a circular shape, but the shape thereof is not limited to the illustrated embodiment.

The first circulation flow path 312 may be a path through which the air introduced through the suction port 311 may flow. The first circulation flow path 312 may be disposed outside the dust collection unit 170. Both ends of the first circulation flow path 312 may be connected to the suction port 311 and the discharge port 313, respectively.

A heater 331 to be described below may be disposed on the first circulation flow path 312. A circulation fan to be described below may be disposed on the first circulation flow path 312. Accordingly, air flowing through the first circulation flow path 312 by the circulation fan may be heated by the heater 331.

For example, the first circulation flow path 312 may be a space including an accommodation space 321a of a fan housing 321 and an internal space of a heater housing 332, which will be described below. As another example, the first circulation flow path 312 may be a space formed between the circulation fan and the heater 331.

Air blown by the circulation fan may flow through the first circulation flow path 312. The air blown by the circulation fan may flow through the first circulation flow path 312 and may be discharged into the internal space S through the discharge port 313.

The discharge port 313 may serve as an outlet for discharging air suctioned through the suction port 311 into the internal space S. The air flowing through the first circulation flow path 312 by the circulation fan may be discharged into the internal space S through the discharge port 313.

The discharge port 313 may be formed at an inner other side of the dust collection unit 170. Specifically, the discharge port 313 may be formed on the other side of the upper portion of the chamber housing 172a.

The discharge port 313 may communicate with a discharge hole 332a formed on a lower surface of the heater housing 332. The air flowing through the first circulation flow path 312 may be discharged into the internal space S through the heater 331, the discharge hole 332a, and the discharge port 313.

The discharge port 313 may be formed to extend in the front-rear direction along one edge of the chamber housing 172a. An open area of the discharge port 313 may be larger than an open area of the suction port 311. A cross-sectional area of the first circulation flow path 312 may increase toward the discharge port 313. Accordingly, when the air heated by the heater 331 is discharged into the internal space S through the discharge port 313, the heated air may be discharged while being widely diffused into the internal space S.

To ensure that the heat discharged from the discharge port 313 may circulate widely in the internal space S, the suction port 311 and the discharge port 313 may be disposed as far apart as possible on the chamber housing 172a. The suction port 311 and the discharge port 313 may be disposed with the dust inlet 173 formed in the chamber housing 172a interposed therebetween. The suction port 311 and the discharge port 313 may be disposed with the dust bag 171a interposed therebetween. The suction port 311 and the discharge port 313 may be disposed at the farthest possible distance from each other on the chamber housing 172a. For example, the suction port 311 and the discharge port 313 may be disposed at diagonal edges of the chamber housing 172a, respectively.

The suction port 311 and the discharge port 313 may be formed on the same surface of the chamber housing 172a. For example, the suction port 311 and the discharge port 313 may be formed on an upper surface of the chamber housing 172a.

The suction port 311 and the discharge port 313 may be formed on side surfaces of the chamber housing 172a, respectively. In this case, the suction port 311 and the discharge port 313 may be formed on the same surface of the chamber housing 172a, but alternatively, may be formed on opposite surfaces so as to face each other. That is, as long as the above functions of the suction port 311 and the discharge port 313 are maintained, the positions at which the suction port 311 and the discharge port 313 are formed are not limited.

The suction port 311 and the discharge port 313 may be formed at the same height from the ground. A penetration direction of the suction port 311 and a penetration direction of the discharge port 313 may be formed parallel to each other. A penetration direction of the dust inlet 173, the penetration direction of the suction port 311, and the penetration direction of the discharge port 313 may be formed parallel to each other.

Meanwhile, a deodorizing filter (not illustrated) may be disposed in the discharge port 313. Accordingly, the air discharged through the discharge port 313 may be sterilized while passing through the deodorizing filter.

An activated carbon filter with excellent odor removal or vapor capture efficiency may be disposed in the discharge port 313. A photocatalytic filter that sterilizes and decomposes various harmful substances may be disposed in the discharge port 313. A plasma and/or ozone filter that filters various contaminants contained in the air may be disposed in the discharge port 313.

The second circulation flow path 314 may serve as a flow path through which the air discharged through the discharge port 313 may flow. The second circulation flow path 314 may be disposed in the internal space. The second circulation flow path 314 may have both ends connected to the suction port 311 and the discharge port 313, respectively.

The second circulation flow path 314 may be at least a portion of the internal space S. The second circulation flow path 314 may be a space in which air flows in the internal space S. The second circulation flow path 314 may be the internal space of the chamber housing 172a and/or the internal space of the dust bag 171a.

The air heated by the heater 331 and discharged into the internal space S may be cooled while circulating through the second circulation flow path 314. The temperature of the air flowing through the second circulation flow path 314 may increase toward the discharge port 313. The air flowing through the second circulation flow path 314 may approach 65 °C toward the discharge port 313. The temperature of the air flowing through the second circulation flow path 314 may decrease toward the suction port 311. The temperature of the air flowing through the second circulation flow path 314 may approach 55 °C toward the suction port 311.

A deodorizing filter (not illustrated) may be disposed on the second circulation flow path 314. The air discharged through the discharge port 313 and flowing through the second circulation flow path 314 may be sterilized while passing through the deodorizing filter.

In addition, an activated carbon filter with excellent odor removal and vapor capture efficiency may be disposed in the second circulation flow path 314. In addition, a photocatalytic filter for sterilizing and decomposing various harmful substances may be disposed in the second circulation flow path 314. In addition, a plasma and/or ozone filter for filtering various contaminants contained in the air may be disposed in the second circulation flow path 314.

The blower 320 may blow air from the suction port 311 toward the discharge port 313.

The blower 320 may include the fan housing 321 and a circulation fan (not illustrated).

The fan housing 321 may be disposed on the chamber housing 172a. The fan housing 321 may have the accommodation space 321a, which accommodates the circulation fan, formed therein.

The accommodation space 321a may communicate with the suction port 311 and the first circulation flow path 312. Alternatively, the accommodation space 321a may be a portion of the first circulation flow path 312.

A lower surface and side surface of the fan housing 321 may each be open. The open lower surface of the fan housing 321 may communicate with the suction port 311, and the open side surface of the fan housing 321 may communicate with the first circulation flow path 312. In this case, penetration directions of the open lower surface of the fan housing 321 and the open side surface of the fan housing 321 may be vertical. In addition, the entire flow path connecting the suction port 311, the accommodation space 321a, and the first circulation flow path 312 may have a single-folded shape, thereby minimizing flow loss in the circulation flow path unit 310.

The circulation fan may be disposed on the circulation flow path unit 310. The circulation fan may be disposed in the accommodation space 321a inside the fan housing 321. The circulation fan may blow air from the suction port 311 toward the discharge port 313 so that the air in the internal space S flows through the first circulation flow path 312.

The circulation fan may be operated after the driving of the dust collection motor 191 is terminated. After the cleaner 200 is seated on the cleaner station 100, when the dust collection motor 191 is driven, dust inside the dust bin 220 of the cleaner 200 may be collected by the dust collection unit 170. In a state in which the dust collection motor 191 is turned off and dust is collected by the dust collection unit 170, the circulation fan may be driven to circulate the air in the internal space S.

The heating unit 330 may heat the air blown by the circulation fan and may include the heater 331 and the heater housing 332.

The heater 331 may be disposed on the circulation flow path unit 310. Specifically, the heater 331 may be disposed on the first circulation flow path 312. The heater 331 may be disposed in front of the circulation fan. The heater 331 may be disposed closer to the discharge port 313 than the circulation fan. A distance between the discharge port 313 and the heater 331 may be smaller than a distance between the discharge port 313 and the circulation fan. Accordingly, the air blown by the circulation fan may be heated while passing through the heater 331, and the heated air may be discharged into the internal space S through the discharge port 313.

Meanwhile, in order to sterilize insects and microorganisms that proliferate in the foreign matter captured in the dust bag 171a, the temperature of the internal space S needs to be maintained at high temperature for a long time. In this case, in order to effectively sterilize insects and microorganisms such as powdery mildew, the insects and microorganisms can be preferably exposed at a temperature of 55 °C or higher for one hour or longer. In addition, the maintenance of the temperature of the internal space S at high temperature for a long time is advantageous for sterilization, but when the foreign substances collected in the dust collection unit 170 contains oil/fat components, there is a risk of ignition when the foreign substances are exposed at a temperature of 65 °C or higher for one hour or longer due to the oil/fat components.

For this reason, the heater 331 may heat the air blown through the discharge port 313 by the circulation fan to 65 °C. That is, since a process in which the air heated by the heater 331 and discharged into the internal space S may be cooled while circulating in the internal space S, and the air circulating in the internal space S is re-suctioned through the suction port 311, heated by the heater 331, and discharged into the internal space S through the discharge port 313 is repeated, the temperature of the internal space S may be maintained at a high temperature close to 65 °C. In addition, when the temperature of the internal space S drops below 55 °C, the heater 331 and the circulation fan re-operate, and thus the temperature of the internal space S may be maintained between 55 °C and 65 °C.

Meanwhile, in order to prevent the heater 331 from operating independently, the heater 331 may heat the air discharged into the internal space S in a state in which the circulation fan is driven. Specifically, when the circulation fan is driven and air behind the circulation fan is blown forward, the blown air may be heated by the heater 331 disposed in front of the circulation fan.

Meanwhile, the heater 331 may be provided in a form in which a heating coil (not illustrated) is wound. Alternatively, the heater 331 may be provided in a form capable of self-heating without including the heating coil. When the heater 331 does not include the heating coil, a height of the heater housing 332 that accommodates the heater 331 can be reduced, thereby increasing space efficiency.

The heater housing 332 may be disposed above the chamber housing 172a. The heater housing 332 may have the accommodation space, which accommodates the heater 331, formed therein. The accommodation space of the heater housing 332 may be at least a portion of the first circulation flow path 312. The accommodation space of the heater housing 332 may communicate with the accommodation space 321a of the fan housing 321.

Accordingly, the suction port 311, the accommodation space 321a of the fan housing 321, the accommodation space 321a of the heater housing 332, the discharge port 313, and the internal space S of the dust collection unit 170 may communicate with each other to form the single circulation flow path 310.

The discharge hole 332a may be formed in the lower surface of the heater housing 332. The discharge hole 332a may be formed to extend in the front-rear direction along one edge of the chamber housing 172a. The discharge hole 332a may communicate with the discharge port 313.

Based on the above embodiment, the airflow circulating through the circulation flow path unit 310 and the internal space S will be described in detail.

First, when the circulation fan begins operating, the air in the internal space S may be suctioned into the accommodation space 321a inside the fan housing 321 through the suction port 311 formed on one side of the upper surface of the chamber housing 172a. Next, the air suctioned into the accommodation space 321a may pass through the circulation fan and flow through the first circulation flow path 312 toward the heater 331. Next, the air blown from the circulation fan may be heated by the heater 331 and discharged back into the internal space S through the discharge port 313. In this case, the heater 331 may heat the blown air to 65 °C. Next, the air discharged into the internal space S may sterilize the inside of the dust bag 171a while circulating in the internal space S by the suction force of the circulation fan. In this case, the temperature of the air circulating in the internal space S may continuously decrease. However, since the air in the internal space S is re-suctioned into the suction port 311 by the circulation fan, heated by the heater 331, and discharged by the circulation fan, the temperature of the air in the internal space S may be as close as possible to 65 °C.

Meanwhile, FIG. 11 shows a block diagram for describing a control configuration of the cleaner system according to the embodiment of the present disclosure.

The control configuration of the cleaner system 10 according to the present disclosure will be described with reference to FIG. 11 as follows.

The cleaner system 10 according to the embodiment of the present invention may further include the controller 400 for controlling the coupling unit 120, the door unit 140, the cover open unit 150, the dust collection unit 170, the suction flow path unit 180, the dust suction module 190, the suction motor 214, the manipulation unit 218, and the battery 240.

The controller 400 may be composed of a printed circuit board and elements mounted on the printed circuit board.

The controller 400 may be classified into a station controller 401 for controlling the cleaner station 100 and a cleaner controller 402 for controlling the cleaner 200. The station controller 401 and the cleaner controller 402 may communicate with each other to exchange information or process data. Hereinafter, unless there is a special limitation, the station controller 401 and the cleaner controller 402 are collectively referred to as the controller 400.

When the coupling sensor 125 detects the coupling of the cleaner 200, the coupling sensor 125 may transmit a signal indicating that the cleaner 200 has been coupled to the coupling unit 120. In this case, the controller 400 may receive the signal of the coupling sensor 125 and determine that the cleaner 200 has been coupled to the coupling unit 120.

In addition, when the charging unit 128 supplies power to the battery 240 of the cleaner 200, the controller 400 may determine that the cleaner 200 has been coupled to the coupling unit 120.

When determining that the cleaner 200 has been coupled to the coupling unit 120, the controller 400 may fix the cleaner 200 by operating the fixing unit motor 133.

When the fixing member 131 or a fixing unit link (not illustrated) moves to a predetermined fixed point, a fixed detection unit 137 may transmit a signal indicating that the cleaner 200 has been fixed. The station controller 401 may receive the signal indicating that the cleaner 200 has been fixed from the fixed detection unit 137 and determine that the cleaner 200 has been fixed. When it is determined that the cleaner 200 has been fixed, the station controller 400 may stop the operation of the fixing unit motor 133.

Meanwhile, when the emptying of the dust bin 220 is finished, the controller 400 may release the fixing of the cleaner 200 by rotating the fixing unit motor 133 in the reverse direction.

When it is determined that the cleaner 200 has been fixed to the coupling unit 120, the controller 400 may open the door 141 of the cleaner station 100 by operating the door motor 142.

The door opening and closing detection unit 144 may transmit a signal indicating that the door 141 has been opened when the door 141 or the door arm 143 reaches a predetermined opening location. The controller 400 may receive the signal indicating that the door 141 has been opened from the door opening and closing detection unit 144 and determine that the door 141 has been opened. When it is determined that the door 141 has been opened, the controller 400 may stop the operation of the door motor 142.

Meanwhile, when the emptying of the dust bin 220 is finished, the controller 400 may close the door 141 by rotating the door motor 142 in the reverse direction.

When it is determined that the door 141 has been opened, the controller 400 may open the discharge cover 222 of the cleaner 200 by operating the cover open motor 152.

The cover opening detection unit 155f may transmit a signal indicating that the discharge cover 222 has been opened when a guide frame 151e reaches a predetermined opening location. The controller 400 may receive the signal indicating that the discharging cover 222 has been opened from the cover open detection unit 155f and determine that the discharging cover 222 has been opened. When it is determined that the discharging cover 222 has been opened, the controller 400 may stop the operation of the cover open motor 152.

The controller 400 may suction the dust inside the dust bin 220 by driving the dust collection motor 191.

The controller 400 may operate the circulation fan to circulate air flowing on the circulation flow path unit 310. After operating the circulation fan, the controller 400 may operate the heater 331.

The controller 400 may operate the heater 331 until the temperature of the air discharged through the discharge port 313 reaches 65 °C. Specifically, the temperature sensor 174 provided on the discharge port 313 or the second circulation flow path 314 may detect the temperature of the internal space S and transmit information on the temperature of the internal space S to the controller 400.

In addition, the controller 400 may receive the temperature information of the internal space S and operate the heater 331 until the temperature of the internal space S reaches 65 °C.

With this configuration, the temperature of the internal space S may be increased to a temperature capable of killing insects and microorganisms present in the internal space S.

Meanwhile, the controller 400 may maintain the temperature of the internal space S at 55 °C or higher and 65 °C or lower for a preset pest control time.

Specifically, the controller 400 may maintain the temperature of the internal space S for a sufficient time to kill insects and microorganisms, including powdery mildew, etc. For example, powdery mildew may be killed by maintaining the temperature of 55 °C or higher and 65 °C or lower for one hour or longer.

Accordingly, the temperature sensor 174 may measure the temperature of the internal space S and transmit the measured temperature to the controller 400, and the controller 400 may re-operate the heater 331 and the circulation fan to reheat the temperature of the internal space S when the temperature of the internal space S drops below 55 °C.

In addition, the controller 400 may measure the time during which the temperature of the internal space S is maintained at 55 °C or higher and 65 °C or lower through a built-in timer (not illustrated), and when the time during which the temperature of the internal space S is maintained at 55 °C or higher and 65 °C or lower is longer than the insecticide time, the reheating by the operation of the heater 331 and the circulation fan may be terminated.

Accordingly, according to the cleaner station 100 of the present invention, it is possible to sterilize insects and microorganisms such as dust mites introduced from the dust bin 220 of the cleaner 200.

Meanwhile, the cleaner station 100 according to the present invention may include the display unit 410.

The display unit 410 may be disposed not only in the housing 110, but also in a separate display device and provided in a terminal such as a mobile phone.

The controller 400 may display a dust bin emptying situation and charging situation of the cleaner 200 by operating a display unit 410.

The display unit 410 may include at least one of a display panel capable of outputting text and/or graphics, and a speaker capable of outputting voice signals and sounds. A user can easily understand a situation, remaining time, and the like of a current ongoing stroke through the information output through the display unit.

Meanwhile, the cleaner station 100 according to the embodiment of the present invention may include a memory 430. The memory 430 may include various data for driving and operating the cleaner station 100.

Meanwhile, the cleaner station 100 according to the embodiment of the present invention may include an input unit 440. The input unit 440 generates key input data input by the user to control the operation of the cleaner station 100. To this end, the input unit 440 may be composed of a key pad, a dome switch, a touch pad (static pressure/electrostatic), etc. In particular, when the touch pad forms a layered structure with the display unit 410, the touch pad may be referred to as a touch screen.

Although the present invention has been described in detail through specific embodiments, this is intended to specifically describe the present invention, and it is apparent that the present invention is not limited thereto, and the present invention can be modified or improved by those skilled in the art without departing from the technical spirit of the present invention.

All simple modifications or changes of the present invention fall within the scope of the present invention, and the specific scope of the present invention will be made clear by the appended claims.

## Claims

1. A cleaner station comprising:
a housing;
a coupling unit disposed in the housing and including a coupling surface to which at least a portion of a cleaner is coupled;
a dust collection unit which is disposed in the housing and collects dust of a dust bin of the cleaner;
a suction flow path unit connecting a dust through hole formed in the coupling surface to the dust collection unit;
a dust collection motor which is accommodated inside the housing and generates a suction force of suctioning the dust inside the dust bin; and
a heat circulation module configured to heat air suctioned from an internal space of the dust collection unit and supplies the heated air to the internal space.

2. The cleaner station of claim 1, wherein the heat circulation module includes:
a circulation flow path unit having a suction port through which the air in the internal space is suctioned and a discharge port that discharges the suctioned air into the internal space;
a circulation fan which is disposed on the circulation flow path unit and blows air from the suction port toward the discharge port; and
a heater configured to heat the air blown by the circulation fan.

3. The cleaner station of claim 2, wherein the dust collection unit includes a dust inlet that guides the air flowing through the suction port into the internal space, and
the suction port and the discharge port are disposed with the dust inlet interposed therebetween.

4. The cleaner station of claim 2, wherein the heater is disposed on the circulation flow path unit, and
a distance from the discharge port to the heater is smaller than a distance from the discharge port to the circulation fan.

5. The cleaner station of claim 2, wherein an open area of the discharge port is larger than an open area of the suction port.

6. The cleaner station of claim 2, wherein the heater heats the air blown through the discharge port up to 65 °C.

7. The cleaner station of claim 2, wherein the temperature of the internal space is 55 °C or higher and 65 °C or lower.

8. The cleaner station of claim 2, wherein the heater heats the air discharged into the internal space in a state in which the circulation fan is driven.

9. The cleaner station of claim 2, wherein the circulation fan is driven after the driving of the dust collection motor is terminated.

10. The cleaner station of claim 2, wherein a deodorizing filter is disposed in the discharge port.

11. The cleaner station of claim 1, wherein the heat circulation module includes:
a suction port through which the air in the internal space is suctioned;
a discharge port through which air is discharged into the internal space;
a first circulation flow path disposed outside the dust collection unit and connecting the suction port to the discharge port;
a second circulation flow path disposed in the internal space and connecting the suction port to the discharge port;
a circulation fan which is disposed on the first circulation flow path and blows air from the suction port toward the discharge port; and
a heater configured to heat the air blown by the circulation fan.

12. The cleaner station of claim 11, wherein a cross-sectional area of the first circulation flow path increases toward the discharge port.

13. The cleaner station of claim 11, wherein a temperature of air flowing through the second circulation flow path increases toward the discharge port.

14. The cleaner station of claim 11, wherein a temperature of air flowing through the second circulation flow path decreases toward the suction port.

15. The cleaner station of claim 11, wherein a deodorizing filter is disposed on the second circulation flow path.
